(19) Europäisches Patentamt
European Patent Office
Office européen des brevets

(11) Numéro de publication: **0 262 053**
**A2**

(12) # DEMANDE DE BREVET EUROPEEN

(21) Numéro de dépôt: **87402141.3**

(22) Date de dépôt: **24.09.87**

(51) Int. Cl.[4]: **C 07 C 103/49**
C 07 C 103/50, A 61 K 31/16,
A 61 K 31/19, A 61 K 31/195

(30) Priorité: **25.09.86 FR 8613413**

(43) Date de publication de la demande:
**30.03.88 Bulletin 88/13**

(84) Etats contractants désignés:
**AT BE CH DE ES FR GB GR IT LI LU NL SE**

(71) Demandeur: **CENTRE NATIONAL DE LA RECHERCHE SCIENTIFIQUE (CNRS)**
**15, Quai Anatole France**
**F-75700 Paris (FR)**

(72) Inventeur: **Fournie-Zaluski, Marie-Claude**
**16 avenue de Bouvines**
**F-75011 Paris (FR)**

**Roques, Bernard**
**12 rue E. Delacroix**
**F-94410 Saint-Maurice (FR)**

(74) Mandataire: **Grosset-Fournier, Chantal Catherine et al**
**SC Ernest Gutmann/Yves Plasseraud 67 boulevard Haussmann**
**F-75008 Paris (FR)**

Une requête en rectification de la description original a été présentée conformément à la règle 88 CBE. Il est statué sur cette requête au cours de la procedure engagée devant la division d'examen (Directives relatives à l'examen pratiqué à l'OEB, A-V, 2.2).

(54) **Nouveaux dérivés d'amino-acides, leur procédé de préparation et compositions pharmaceutiques les contenant.**

(57) L'invention a pour objet des composés de formule générale :

```
    OR  O
    |   ||
H-N-  C-CH2-CH-A-B-CH-CH-COR4
            |      |  |
            R1     R2 R3
```

dans laquelle
R peut représenter un atome d'hydrogène ;
$R_1$ peut représenter un groupe alcoyle saturé, linéaire ou ramifié, comportant de 1 à 10 atomes de carbone ;
$R_2$ et $R_3$ sont identiques ou différents et représentent :
- un atome d'hydrogène,
- un groupe alcoyle saturé, linéaire ou ramifié, de 1 à 10 atomes de carbone ;

$R_4$ peut représenter un groupe hydroxyle ;
A-B représente un groupe amide CO-NH ou rétro amide NH-CO.

Application à la préparation de compositions pharmaceutiques.

EP 0 262 053 A2

## Description

### NOUVEAUX DERIVES D'AMINO-ACIDES, LEUR PROCEDE DE PREPARATION ET COMPOSITIONS PHARMACEUTIQUES LES CONTENANT

L'invention a pour objet de nouveaux dérivés d'amino-acides, leur procédé de préparation.

L'invention est également relative à de nouveaux médicaments, contenant à titre de principe actif, ces nouveaux amino-acides, ainsi que leurs sels physiologiquement acceptables.

Dans le texte, le terme médicament désigne une composition pharmaceutique qui contient l'un au moins des nouveaux dérivés d'amino-acides, en association avec un véhicule pharmaceutiquement acceptable.

On sait que les enképhalines sont dégradées par trois enzymes appartenant à la classe des métallopeptidases, l'endopeptidase neutre EC 3.4.24.11 (ou enképhalinase) qui coupe la liaison $Gly^3$-$Phe^4$ des enképhalines, une aminopeptidase qui coupe la liaison $Tyr^1$-$Gly^2$ et enfin une dipeptidylaminopeptidase (enképhalinase B) qui coupe entre les deux glycines.

On connait déjà des dérivés de dipeptides ayant une activité inhibitrice sur l'enképhalinase. On peut par exemple citer les composés qui sont l'objet du demande de brevet nº 2 518 088.

On sait également que l'aminopeptidase est inhibée de façon non sélective par la bestatine (Suda H, T. Aoyagi, T. Takeushi and H. Umezawa, Arch. Biochem. Biophys. 117, 196-200 (1976)) et certains de ces dérivés.

Mais, aucun inhibiteur spécifique de la dipeptidylaminopeptidase n'est à ce jour connu. Or, la Société demanderesse a synthétisé de nouveaux composés, lesquels sont susceptibles d'inhiber conjointement ces trois activités enzymatiques.

L'un des aspects de l'invention est de fournir de nouveaux composés, susceptibles d'inhiber conjointement les enzymes de dégradation des enképhalines, et pouvant prolonger les effets des enképhalines endogènes ou potentialiser l'action d'analogues synthétiques administrés de façon exogène. Ces composés sont susceptibles de remplacer les agents morphiniques dans toutes leurs proptiétés sans en avoir les graves inconvénients, en particulier au niveau des phénomènes d'accoutumance et de dépendance.

L'un des aspects de l'invention a pour objet de fournir de nouveaux composés doués de propriétés pharmacologiques intéressantes, doués en particulier de propriétés inhibitrices des enzymes de dégradation des enképhalines.

L'un des aspects de l'invention est donc de fournir de nouveaux dérivés analogues de dipeptides, présentant des propriétés analgésiques supérieures à celles induites par des inhibiteurs sélectifs de l'enképhalinase ou de l'aminopeptidase.

L'un des aspects de l'invention est de fournir de nouvelles compositions pharmaceutiques, efficaces notamment dans le traitement des algies et des douleurs.

Les composés de l'invention répondent à la formule I

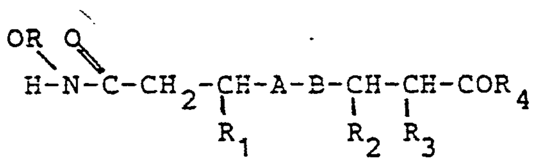

(I)

dans laquelle

- R est
- un atome d'hydrogène,
- un groupe acyle aliphatique, linéaire ou ramifié, de 2 à 11 atomes de carbone et de préférence de 2 à 5 atomes de carbone, dans lequel 1 atome d'hydrogène peut être substitué par
  * un halogène, notamment le fluor, ou un groupe trihalogénométhyle,
  * un groupe $OR_5$, $R_5$ représentant l'hydrogène, un groupe alcoyle de 1 à 6 atomes de carbone ou un groupe acyle, de 2 à 7 atomes de carbone, lesquels groupes alcoyle ou acyle sont peuvent être cyclisés, aliphatiques ou aromatiques ;
- un groupe benzoyle ou phényle acétyle, pouvant comporter sur le cycle aromatique
  * 1 à 5 atomes d'halogène, notamment de fluor,
  * un groupe $OR_5$, $R_5$ représentant l'hydrogène ou un groupe alcoyle, de 1 à 6 atomes de carbone, ou un groupe acyle, de 2 à 7 atomes de carbone, lesquels groupes alcoyle ou acyle peuvent être cyclisés, aliphatiques ou aromatiques ;
  * un groupe nitro,
  * un groupe amino, éventuellement mono ou disubstitué par un radical alcoyle aliphatique ou cyclisé de 1 à 6 atomes de carbone ;
- un groupe α-amino acylé

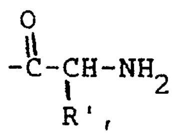

dans lequel
R′ représente le radical benzyle, p-hydroxybenzyle, guanidino-3 propyle ou amino-4-butyle,

$R_1$ représente
- un groupe alcoyle saturé, linéaire ou ramifié, comportant de 1 à 10 atomes de carbone, notamment de 1 à 6 atomes de carbone ;
- un groupe methyl cycloalkyle de 5 à 6 atomes de carbone, un groupe benzyle, les groupes benzyle ou méthyl cycloalcoyle, comportant éventuellement
* 1 à 5 atomes d'halogène, notamment de fluor,
* un hydroxyle,
* un groupe alcoxy, linéaire ou ramifié, comportant de 1 à 4 atomes de carbone
* un groupe amino, éventuellement mono ou disubstitué par un groupe alcoyle aliphatique de 1 à 6 atomes de carbone, éventuellement cyclisé ;

$R_2$ et $R_3$ sont identiques ou différents et représentent
- un atome d'hydrogène ;
- un groupe alcoyle saturé, linéaire ou ramifié, de 1 à 10 atomes de carbone, notamment de 1 à 6 atomes de carbone,
- un groupe phényle ou benzyle, pouvant comporter
* 1 à 5 atomes d'halogène, notamment de fluor,
* un groupe $OR_5$, $R_5$ représente l'hydrogène ou un groupe alcoyle linéaire ou ramifié, de 1 à 5 atomes de carbone, ou un groupe acyle, linéaire ou ramifié de 2 à 5 atomes de carbone,
* un groupe alcoyle aliphatique, linéaire ou ramifié comportant de 1 à 4 atomes de carbone ;

$R_2$ et $R_3$ peuvent être des chaînes alcoyle saturées aliphatiques comportant de 1 à 10 atomes de carbone, notamment de 1 à 6 atomes de carbone, ou des chaînes alcoyle insaturées, comportant de 2 à 10 atomes de carbone, notamment de 2 à 6 atomes de carbone, dans lesquelles 1 ou 2 atomes de carbone peuvent être substitués par des atomes d'oxygène, de soufre ou d'azote, pouvant conduire à la formation de cycles hydrocarbonés saturés ou aromatiques ou de 5 ou 6 maillons, ou d'hétérocycles saturés ou aromatiques de 5 ou 6 maillons,

$R_4$ représente
- un groupe hydroxyle,
- un groupe alcoxy aliphatique, linéaire ou ramifié, comportant de 1 à 20, avantageusement de 1 à 8, et de préférence de 1 à 4 atomes de carbone, saturé ou insaturé, dans lequel 1 atome de carbone peut être substitué par
* un atome d'halogène, notamment de fluor ou un groupe trihalogénométhyle ;
* un groupe hydroxy,
* un groupe amino, pouvant être mono ou disubstitué par un groupe alcoyle aliphatique de 1 à 6 atomes de carbone, éventuellement cyclisé

* par un groupe aminoxyde 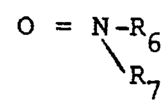

dans lequel $R_6$ et $R_7$ représentent un groupe alcoyle aliphatique de 1 à 6 atomes de carbone, éventuellement cyclisé,
- un groupe $-O-(CH_2)_n-R_5$, n allant de 1 à 4, $R_5$ représentant un cycle saturé de 4 à 6 atomes de carbone ;
- un groupe phényloxy ou phénylalcoyloxy de 1 à 4 atomes de carbone, dans lequel le noyau phényle comporte éventuellement
* 1 à 5 atomes d'halogène, notamment de fluor ;
* un groupe hydroxy,
* un groupe alcoxy, dans lequel le radical alcoyle comporte de 1 à 4 atomes de carbone,
* trihalogénomethyle ;
* un groupe nitro, ou amino ;
- un groupe amino, non substitué ou mono ou disubstitué
* par un groupe alcoyle de 1 à 8 atomes de carbone, linéaire ou ramifié,
* par un groupe hydroxyalcoylé de 1 à 8 atomes de carbone,
* par un phényle ou phénylalcoyle dans lequel le groupe alcoyle comporte de 1 à 4 atomes de carbone,

- un reste aminoacide 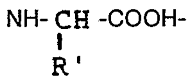

dans lequel R′ représente le radical benzyle, p.hydroxybenzyle, guanidino-3 propyl et amino-4 butyle ; A-B représente un groupe amide CO-NH ou retro amide NH-CO.

Les composés de formule I ont 1, 2 ou 3 atomes de carbone asymétrique. Ils existent donc sous forme de mélange racémique, sous forme d'énantiomères, de diastéréoisomères ou de stéréoisomères.

L'invention a également pour objet les sels d'addition des composés de formule I, obtenus avec des acides organiques ou minéraux physiologiquement acceptables.

A titre de sels organiques ou minéraux physiologiquement acceptables, on peut citer par exemple le chlorhydrate, le bromhydrate, les sulfates, les nitrates, les borates, les phosphates, le méthane sulfonate, l'acétate, le fumarate, le succinate, l'ascorbate, l'oxalate, le lactage, le pyruvate, le citrate, le tartrate, le maléate, le malonate, le benzoate, le salicyclate, le dichloro-2,6 benzoate, le triméthoxy benzoate, le diaminobenzène sulfonate, le chromoglycate, le benzène sulfonate, le cyclohexane sulfonate, le toluène sulfonate, le dipropyl acétate, le glucose-1 phosphate.

Une classe avantageuse de composés de l'invention est constituée par ceux dans lesquels A-B représente CO-NH, et répondant à la formule $I_A$ suivante

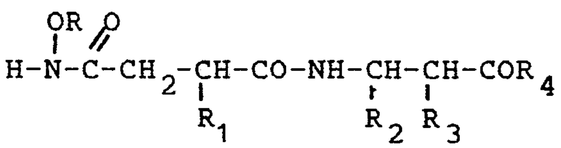

$(I_A)$

dans laquelle R, $R_1$, $R_2$, $R_3$ et $R_4$ ont les significations précédemment indiquées. Ce groupe sera dans la suite désigné par groupe $I_A$.

Une autre classe avantageuse des composés de l'invention est constituée par ceux dans lesquels A-B représentent NH-CO, et répondant à la formule suivante

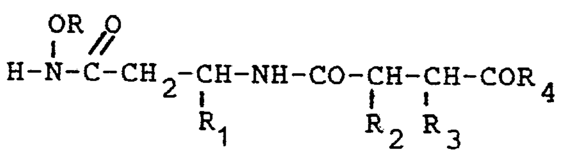

$(I_B)$

dans laquelle R, $R_1$, $R_2$, $R_3$ et $R_4$ ont les significations précédemment indiquées.

Ce groupe sera dans la suite désigné par groupe $I_B$.

Une autre classe avantageuse des composés de l'invention est constituée par ceux dans lesquels $R_1$ est choisi parmi

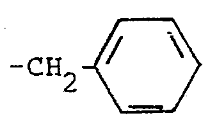

ou

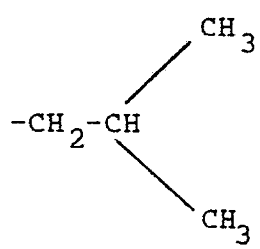

Ce groupe sera dans la suite désigné par groupe $I_C$.

Une autre classe avantageuse des composés de l'invention est constituée par ceux appartenant aux groupes $I_A$, $I_B$, $I_C$ et dans lesquels R est choisi dans le groupe constitué par l'hydrogène,

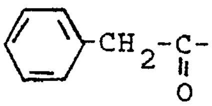 ou 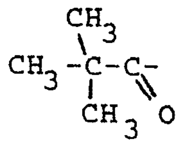

Ce groupe sera dans la suite désigné par groupe $I_D$.

Une autre classe avantageuse des composés de l'invention est constituée par ceux appartenant aux groupes $I_A$, $I_B$, $I_C$, $I_D$ et dans lesquels $R_4$ représente un groupe hydroxy, ou un groupe alcoxy aliphatique, linéaire ou ramifié, comportant de 1 à 20, avantageusement de 1 à 8, et de préférence de 1 à 4 atomes de carbone, saturé ou insaturé, dans lequel 1 à 3 atomes de carbone peuvent être substitués par un groupe hydroxy.

Ce groupe sera dans la suite désigné par groupe $I_E$.

Une autre classe avantageuse des composés de l'invention est constituée par ceux appartenant aux groupes $I_A$, $I_B$, $I_C$, $I_D$, $I_E$ et dans lesquels l'un au moins des substituants $R_2$ et $R_3$ est différent de l'hydrogène.

Ce groupe sera dans la suite désigné par groupe $I_F$.

Une autre classe avantageuse des composés de l'invention est constituée par ceux appartenant aux groupes $I_A$ et $I_B$, dans lesquels $R_2$ et $R_3$ sont choisis dans le groupe constitué par H, $CH_3$,

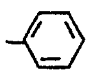

ou $-CH_2-$, ou bien $R_2$ et $R_3$ forment un cycle aliphatique saturé à 5 et 6 chaînons avec les deux carbones auxquels ils sont respectivement fixés sur la chaîne

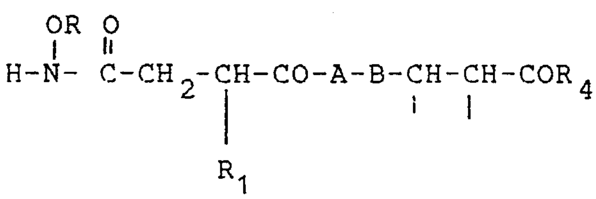

Ce groupe sera dans la suite désigné par groupe $I_G$.

Une autre classe avantageuse de produits selon l'invention est constituée par ceux appartenant aux groupes $I_A$, $I_B$, $I_C$, $I_D$, $I_E$, $I_F$ et $I_G$, et dans lesquels la configuration du premier carbone asymétrique est (R).

Un groupe avantageux de composés selon l'invention est constitué par ceux de formule suivante :

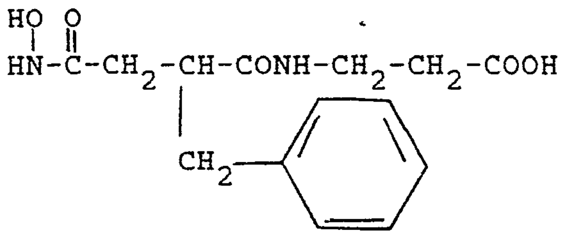

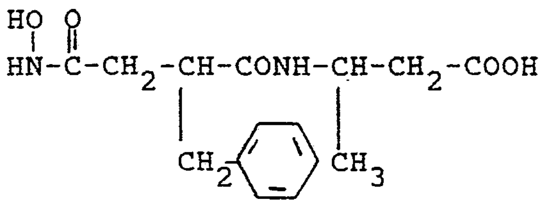

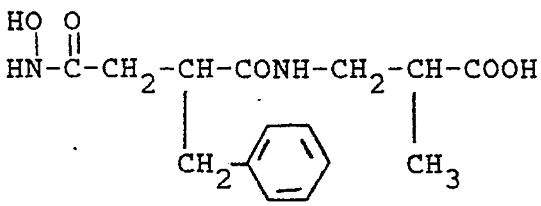

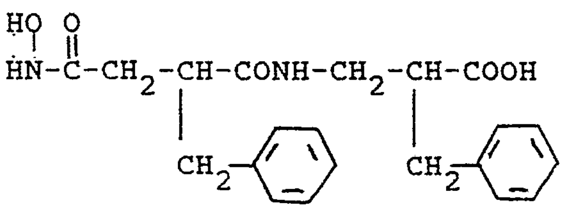

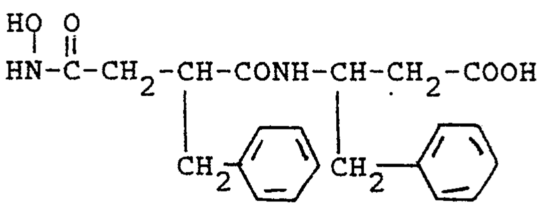

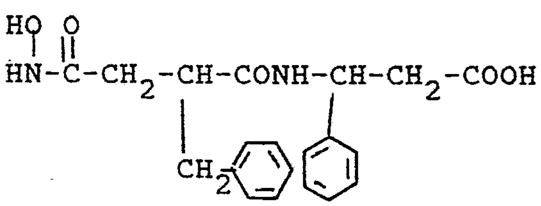

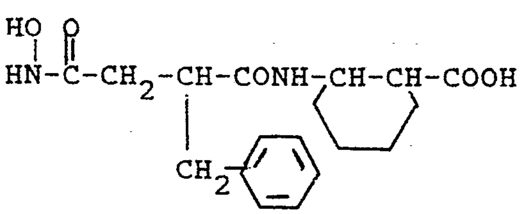

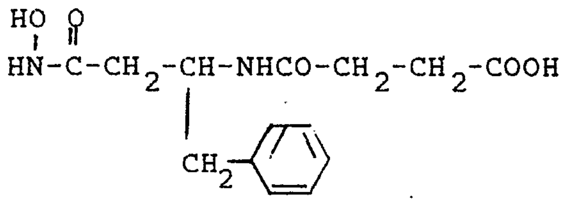

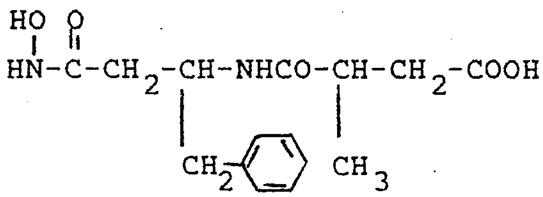

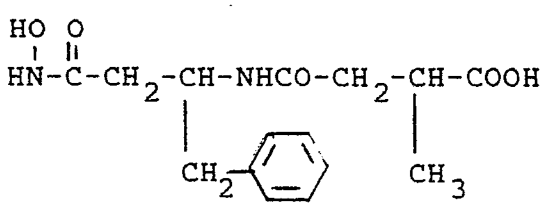

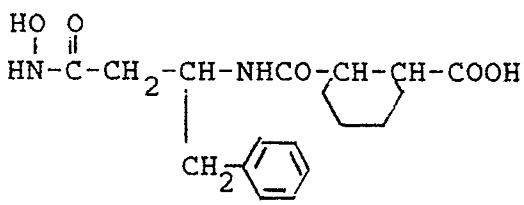

Les composés de l'invention peuvent être préparés comme indiqué ci-après.

Les composés de formule $I_A$, dans lesquels A-B représente une liaison peptidique CONH peuvent être obtenus par exemple en mettant en oeuvre le procédé suivant :

- on fait réagir le composé de formule II

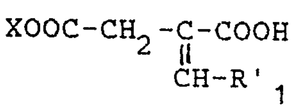

(II)

dans laquelle X représente un groupe alcoyle de 1 ou 2 atomes de carbone, notamment $C_2H_5$, et $R'_1$ représente

- un groupe alcoyle saturé linéaire ou ramifié comportant de 1 à 10 atomes de carbone, notamment de 1 à 5 atomes de carbone ou un groupe alcoyle insaturé linéaire de 2 à 9 atomes, notamment de 2 à 5 atomes de carbone ;
- un groupe cycloalkyle de 5 à 6 atomes de carbone, un groupe phényle, les groupes phényle ou cycloalcoyle, comportant éventuellement
  * 1 à 5 atomes d'halogène, notamment de fluor,
  * un hydroxyle,
  * un groupe alcoxy, linéaire ou ramifié, comportant de 1 à 4 atomes de carbone
  * un groupe amino, éventuellement mono ou disubstitué par un groupe alcoyle aliphatique de 1 à 6 atomes de carbone, éventuellement cyclisé ;

sur le composé de formule III

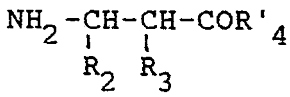

(III)

dans lequel :
R'₄ représente un groupe amide ou un ester tertiobutylique,
$R_2$ et $R_3$ ont les significations indiquées précédemment,
pour obtenir le composé de formule IV

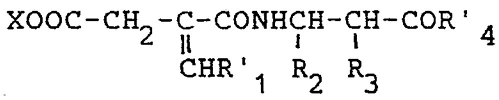

(IV)

dans les conditions classiques de synthèse peptidique, par exemple en utilisant la dicyclohexylcarbodiimide et l'hydroxybenzotriazole,
- on transforme ensuite le composé IV obtenu à l'étape précédente, notamment par hydrolyse alcaline, en composé de formule V

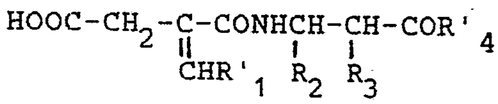

(V)

- on condense ensuite le composé de formule V obtenu précédemment sur le composé de formule VI

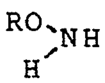

(VI)

dans lequel R a la signification précédemment indiquée, pour obtenir le composé de formule VII

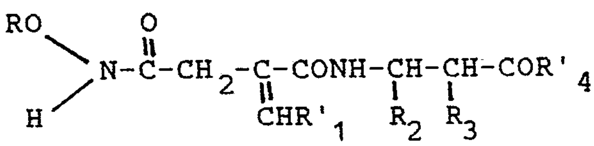

(VII)

dans les conditions classiques de synthèse peptidique, par exemple en utilisant la dicyclohexylcarbodiimide et l'hydroxybenzotriazole,
- puis, on effectue une hydrogénation catalytique sur le composé VII obtenu à l'étape précédente, pour obtenir le composé de formule $I'_A$

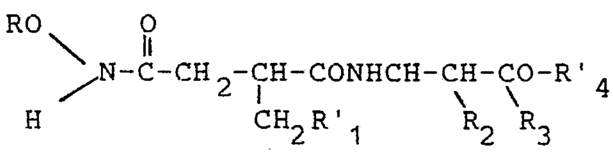

($I'_A$)

Le composé de formule $I'_A$ correspond au composé de formule $I_A$, dans le cas où $R_4$ a la même signification que $R'_4$, c'est-à-dire représente un groupe amide ou un ester tertiobutylique.

Pour obtenir un composé de formule $I_A$

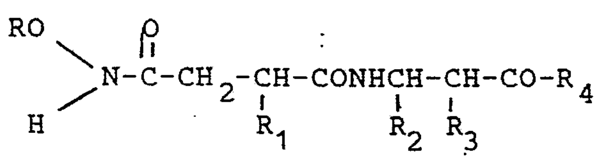

($I_A$)

dans laquelle $R_4$ est différent de $R'_4$, c'est-à-dire $R_4$ est différent d'un groupe amide et d'un ester tertiobutylique,

* soit on soumet le composé de formule VII

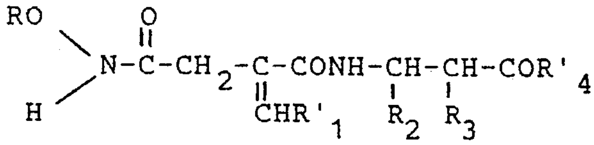

(VII)

dans lequel $R'_4$ représente un ester tertiobutylique, à une hydrolyse acide, pour obtenir le composé de formule VIIbis, indiquée ci-après, dans lequel $R_4$ = OH,

* soit on soumet le composé de formule VII

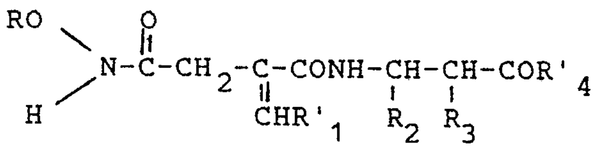

(VII)

dans lequel $R'_4$ représente un ester tertiobutylique, à une hydrolyse acide, suivie d'une reestérification pour obtenir le composé de formule VII bis

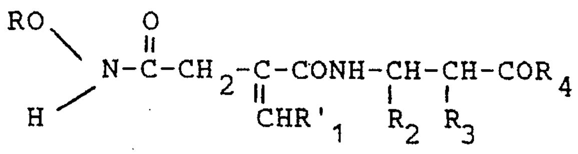

(VII bis)

puis on soumet le composé VII bis obtenu ci-dessus, à une hydrogénation catalytique pour obtenir le composé de formule $I_A$

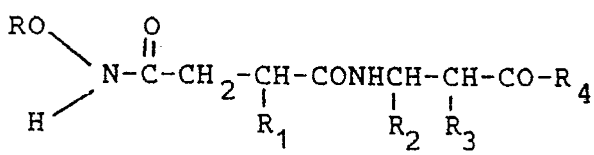

$(I_A)$

* soit on soumet le composé de formule $I'_A$

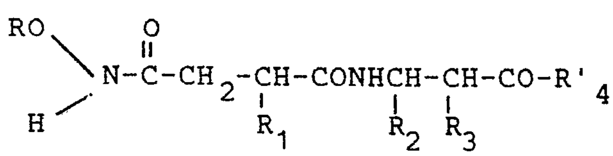

$(I'_A)$

dans lequel $R'_4$ représente un ester tertiobutylique, à une hydrolyse acide pour obtenir le composé de formule $I_A$ dans lequel $R_4$ = OH,

* soit on soumet le composé de formule $I'_A$

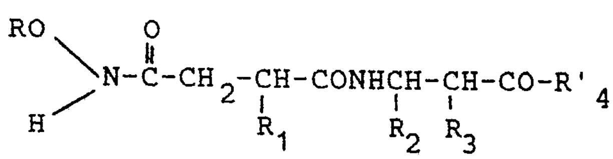

$(I'_A)$

dans lequel $R'_4$ représente un ester tertiobutylique, à une hydrolyse acide suivie d'une reestérification pour obtenir le composé de formule $I_A$

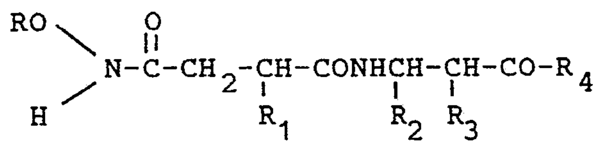

($I_A$)

Les composés de formule II sont obtenus par action de l'éthanol sur les anhydrides cycliques correspondants dont la préparation est bien connue de l'homme de l'art, à savoir condensation de Stobbe entre un aldéhyde $R'_1$-CHO et le succinate d'éthyle, suivie d'une hydrolyse alcaline et d'une deshydratation intramoléculaire.

En ce qui concerne les composés de formule III, certains sont disponibles dans le commerce.

Les composés de formule III, pour lesquels $R_3$ représente H, peuvent être obtenus par réaction de Arndt-Eistert, à partir d'α-aminoacides, suivi d'une esterification ou amidification selon le schéma réactionnel suivant :

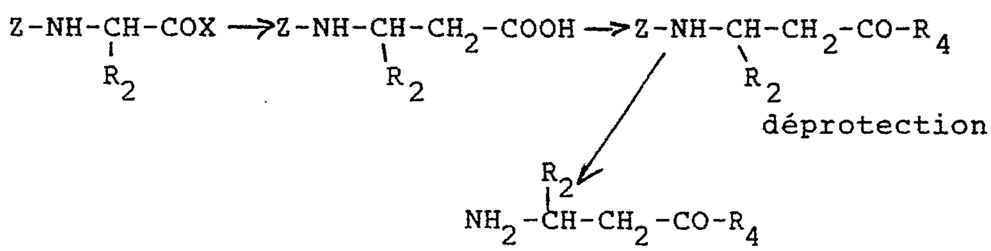


X représentant un atome d'halogène, notamment Cl, ou

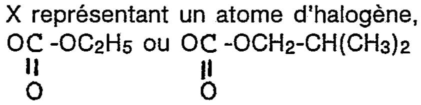
ou

$R_2$ et R4 ayant les significations précédemment indiquées, Z étant un groupement protecteur des fonctions amines, par exemple le groupe t-butyloxycarbonyl (BOC)ou benzyloxycarbonyle.

Les composés III peuvent également être obtenus par réduction de succinamides par une réaction de type "dégradation d'Hofmann", suivant le schéma réactionnel :

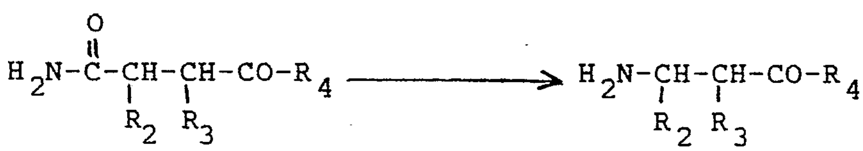

$R_2$, $R_3$ et $R_4$ ayant les significations ci-dessus indiquées.

Les composés III pour lesquels $R_2$ représente H, peuvent être obtenus

* par addition d'un excès d'hydroxylamine sur un dérivé insaturé, selon le schéma réactionnel suivant :

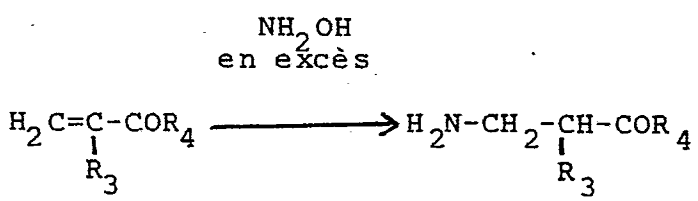


$R_3$ et $R_4$ ayant les significations indiquées ci-dessus

* ou à partir de dérivés monohalogénés, en substituant l'halogène par un groupe nitrile -C≡N, suivi d'une hydrogénation catalytique selon le schéma reactionnel suivant :

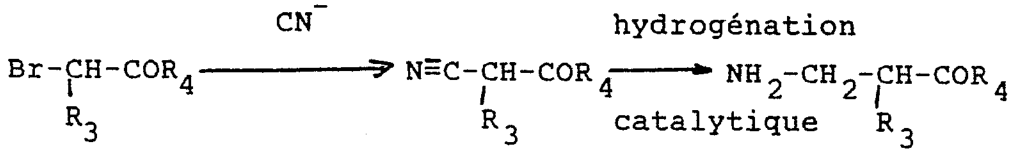


$R_3$ et $R_4$ ayant les significations ci-dessus indiquées.

Les composés de formule générale $I_B$, dans lesquels A-B représente une liaison NH-CO (rétropeptidique), peuvent être obtenus en mettant en oeuvre le procédé suivant

- on fait réagir le composé de formule

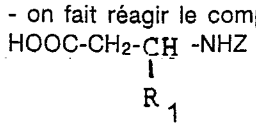 (VIII)

dans lequel $R_1$ a la signification précédemment indiquée et Z représente le groupe t-butyloxycarbonyl (BOC) ou benzyloxycarbonyle sur le composé de formule

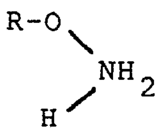

(VI)

R ayant la signification ci-dessus indiquée, dans les conditions peptidiques classiques, notamment en utilisant de la dicyclohexylcarbodiimide et de l'hydroxybenzotriazole, pour obtenir le composé de formule IX

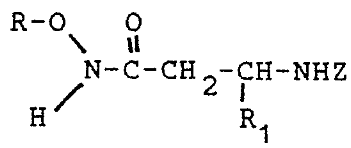

(IX)

- puis on déprotège l'amine en éliminant le groupe Z, par exemple à l'aide d'acide trifluoroacétique, lorsque Z représente BOC et à l'aide d'hydrogénation catalytique lorsque Z représente le groupe benzyloxycarbonyle, pour obtenir le composé de formule X

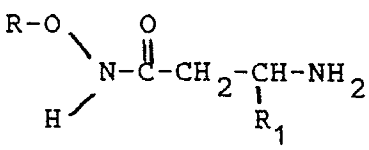

(X)

- puis on fait réagir sur le composé le composé de formule XI ci-après indiqué

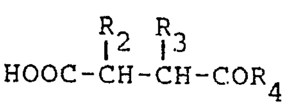

(XI)

$R_2$, $R_3$ et $R_4$ ayant les significations ci-dessus indiquées,
dans les conditions de synthèse peptidique, classiques notamment à l'aide de dicyclohexylcarbodiimide et d'hydroxybenzotriazole, pour donner le composé de formule IB

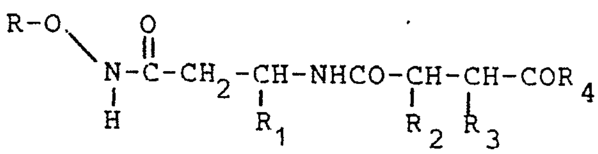

$(I_B)$

Les β-aminoacides protégés VIII sont obtenus comme décrit ci-dessus à partir d'α-aminoacides.

Les monoesters suciniques XI sont obtenus par monosaponification de diesters commerciaux ou synthétisés par différentes méthodes.

Les monoesters succiniques XI peuvent être avantageusement obtenus par la condensation entre un α bromester ou un dérivé et un diester malonique, en présence d'une base forte puis en hydrolysant

sélectivement en milieu basique les fonctions esters COOR', puis en effectuant une décarboxylation, par chauffage selon le schéma réactionnel suivant :

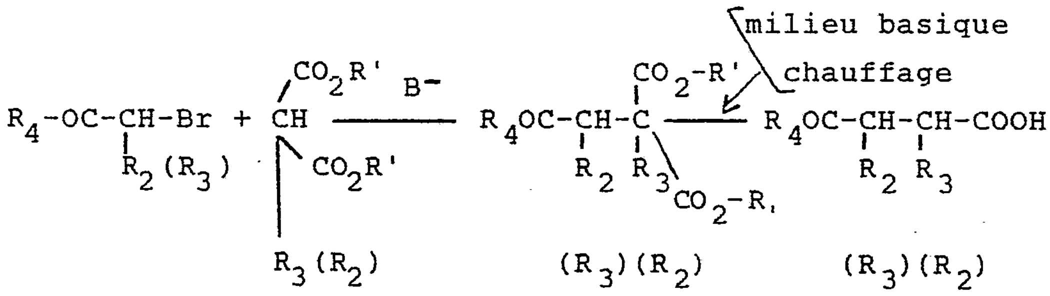


B- représentant une base forte,
R' représentant $CH_3$ ou $C_2H_5$,
$R_2$, $R_3$ et $R_4$ ayant les significations indiquées ci-dessus.

Les monoesters succiniques XI peuvent également être obtenus par alcoylation sélective de monoesters d'acides succiniques substituées (J. Org. Chem., 37, 4, 1972).

Le schéma réactionnel peut s'écrire de la façon suivante :

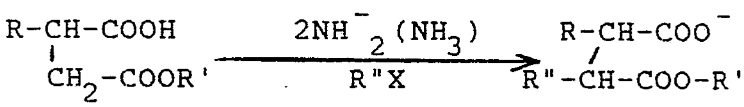


R représentant $R_2$ ou $R_3$,
R' représentant un méthyl, éthyl, benzyle ou terbutyle,
R" représentant $R_2$ ou $R_3$,
X représentant un halogène, notamment Cl ou Br.

Les monoesters succiniques XI peuvent également être obtenus par condensation d'ester d'énolates en présence de sels de cuivre (JACS 93, 4605, 1971).

Les monoesters succiniques XI peuvent être également obtenus par réaction d'α-lithiocarboxylates ou d'esters d'enolates avec des esters α-halogénés ou des carboxylates α-halogénés de lithium (Synthesis, 1980, 710).

En ce qui concerne les sels d'addition des composés de l'invention, on les forme par réaction avec des bases telles que NaOH ou par réaction d'échange d'ions.

On forme les sels d'une manière classique en faisant réagir la forme libre du produit avec un ou plusieurs équivalents de la base ou de l'acide approprié fournissant l'anion ou le cation désiré dans un solvant ou un milieu dans lequel le sel est insoluble ou dans l'eau et en éliminant l'eau par lyphilisation. En neutralisant le sel par un acide insoluble comme une résine échangeuse de cations sous forme hydrogène [par exemple la résine polystyrène-acide-sulfonique Dowex 50 (Mikes, Laboratory Handbook of Chromatographic Methods (Van Nostrand, 1961), page 256], en éluant avec un tampon volatil (par exemple pyridine/acide acétique) et en extrayant avec un solvant organique, on peut obtenir la formule libre et, si on le désire, on peut former un autre sel.

Les exemples suivants sont relatifs à l'obtention de composés de l'invention et sont donnés à titre illustratif et non limitatif.

Exemple 1

Etape 1

Ester t-butylique de la N[benzylidène-2 ethoxycarbonyl-3 oxo-1 propyl] β-alanine

A une solution de 4 g d'acide éthoxycarbonyl-3 benzylidène-2 propanoique dans 100 ml de THF refroidie à 0°C, on ajoute successivement une solution de 2,50 g de l'ester t-butylique de la β-alanine dans 70 ml de $CHCl_3$, une solution de 2,6 g d'hydroxybenzotriazole dans 50 ml de THF et 3,56 g de dicyclolexylcarbodiimide dans 50 ml de $CHCl_3$.

Après une nuit à température ordinaire, le précipité de dicyclohexyl-urée (DCU) est filtré et les solvants évaporés à sec. Le résidu est repris dans l'acétate d'éthyle et est lavé successivement par de l'eau (50 ml), une solution à 10 % d'acide citrique (3 x 50 ml), de l'eau (50 ml), une solution saturée de $NaHCO_3$ (3 x 50 ml) de l'eau (50 ml) et enfin une solution saturée de NaCl (50 ml). La phase organique est séchée sur $Na_2SO_4$ et évaporée à sec.

On obtient 6,2 g (98 %) d'un produit huileux.

Rf ($CHCl_3/CH_3OH/CHCOOH$ = 9/1/0,5) = 0.66.

Etape 2

Ester t-butylique de la N[benzylidène-2 carboxy-3 Oxo-1 propyl] β-alanine

A une solution de 3,5 g du composé précédent dans 80 ml d'un mélange éthanol/eau (2.1) sont ajoutés 10,7 ml d'une solution de NaOH 1N. Après 4 heures à température ambiante, on évapore à sec, reprend par l'eau, lave à l'acétate d'éthyle (40 ml), acidifie par HCl 1N à pH 2 et extrait à l'acétate d'éthyle. La phase organique est lavée à l'eau et avec une solution saturée de NaCl, puis séchée sur $Na_2SO_4$.

Après évaporation à sec, on obtient 16 g d'un solide jaune pâle (Rdt 50 %)
Rf ($CHCl_3/CH_3OH/CH_3COOH$ = 9/1/0.5) = 0,58.

Etape 3

Ester t-butylique de la N[Benzyloxyaminocarbonyl-3 benzylidène-2 oxo-1 propyl] β-alanine

A une solution de 1,5 g du composé précédent dans 30 ml de THF, sont ajoutées successivement une solution de 0,72 g de chlorhydrate de benzylhydroxylamine et de 640 μl de triethylamine dans 20 ml de $CHCl_3$, une solution de 0,7 g d'hydroxybenzotriazole dans 10 ml de THF et 0,94 g de DCC dans 10 ml de $CHCl_3$.

Après une nuit à température ordinaire, la réaction est traitée comme dans l'exemple 1.
On obtient 0,84 g (Rdt 42 %) d'une solide jaune pale, recristallisable dans l'éther.
Rf ($CHCl_3/MeOH$ = 9/1) 0,46.

Etape 4

N[N(Benzyloxy) amino-carbonyl-3 benzylène-2 oxo-1 propyl] β-alanine

On solubilise 0,37 g du composé précédent dans 1,0 ml de $CH_2Cl_2$ et on ajoute à 0°C 1,2 ml de d'acide trifluoroacétique. On laisse une nuit à 0°C. On évapore à sec sous vide et reprend par l'éther.

On obtient un précipité blanc 0,20 g (Rdt 62 %) F 114° C - Rf ($CHCl_3/CH_3OH/CH_3COOH$ = 9/1/0,5) 0,51.

Etape 5

N[N(hydroxy) amino-carbonyl-3 benzyl-2 Oxo-1 propyl] β-alanine

Une solution de 0,20 g du composé précédent dans 10 ml de $CH_3OH$ est hydrogénée à température et à pression ordinaire en présence de 32 mg de Pd/C. Après filtration du catalyseur, on évapore à sec le solvant et on obtient un solide blanc = 0,102 g (Rdt 58 %)
F = 60°C - Rf ($CHCl_3/CH_3OH/CH_3COOH$ = 9/1/0,5) = 0,25.
Le composé obtenu est un mélange des deux enantiomères.

**Exemple 2**

Etape 1

Ester t-butylique de la N[benzylidène-2 ethoxycarbonyl-3 oxo-1 propyl] methyl-3 β-alanine

Ce composé est obtenu en suivant le procédé utilisé pour le composé 1, étape 1, à partir de 1,3 g de l'acide benzylidène-2 ethoxycarbonyl-4 propanoïque et 0,94 g de l'ester t-bytylique de la méthyl-3 β alanine.
On obtient un composé huileux 2,15 g (Rdt 94 %)
Rf ($CHCl_3/CH_3OH$ = 9/1) 0,70.

Etape 2

Ester t-butylique de la N[benzylidène-2 carboxy-3 oxo-1 propyl] methyl-3 β-alanine

Ce composé est obtenu en suivant le procédé décrit dans l'exemple 1, étape 2. A partir de 2,15 g du composé précédent on obtient 1,1 g (55 %) d'un produit huileux
Rf ($CHCl_3/CH_3OH/CH_3COOH$ = 9/1/0,5) 0,62.

Etape 3

Ester t-butylique de la N[benzyloxycarbonyl-3 benzylidène-2 oxo-1 propyl] methyl-3 β alanine

Le composé est obtenu en suivant le procédé décrit dans l'exemple 1, étape 3. A partir de 1,1 g du composé précédent, on obtient 19 (75 %) d'un produit solide jaune pâle purifié par chromatographie sur gel de silice $CH_2Cl_2/(C_2H_5)_2O$ = 9/1) Rf ($CHCl_3/CH_3OH$ = 9/1) 0,57.

Etape 4

N[N(benzyloxy) amino carbonyl-3 benzylidène-2 oxo-1 propyl] methyl-3 β alanine

Ce composé est obtenu en suivant le procédé utilisé dans l'exemple 1, étape 4. A partir de 0,88 g du composé précédent, on obtient 0,48 g d'un solide blanc (Rdt 62 %) F = 134°C - Rf ($CHCl_3/CH_3OH/CH_3COOH$ = 9/1/0,5) 0,65.

Etape 5

N[N(hydroxy)amino carbonyl-3 benzyl-2 oxo-1 propyl] methyl-3 β alanine

A partir de 0,48 g du composé précédent, on obtient, ne utilisant le procédé utilisé dans l'exemple 1, étape 5, 0,26 g d'un solide blanc (Rdt 70 %)
F = 114°C - Rf ($CHCl_3/CH_{34}OH/CH_3COOH$ = 9/1/0,5) 0,36. Le composé est obtenu sous forme des 4 isomères, lesquels peuvent être séparés, notamment par chromatographie.

**Exemple 3**

Etape 1

Ester t-butylique de la N[benzylidène-2 ethoxycarbonyl-3 Oxo-1 propyl] methyl-2 β alanine

Ce composé est obtenu à partir de 3,46 g d'acide ethoxycarbonyl-3 benzylidène-2 propanoïque et de 2,35 g d'ester t-butylique de la méthyl-2 β alanine en suivant le procédé décrit dans l'exemple 1, étape 1.
On obtient 5,45 g d'une huile jaune (Rdt 98 %)
Rf ($CHCl_3/CH_3OH$ = 9/1) 0,76.

Etape 2

Ester t-butylique de la N[benzylidène-2 carboxy-3 Oxo-1 propyl] méthyl-2 β alanine

Ce composé est obtenu à partir de 5,2 g du composé précédent en suivant le procédé décrit dans l'exemple 1, étape 2. On obtient 4,5 g d'un produit huileux (Rdt 92%)
Rf ($CHCl_3/CH_3OH/CH_3COOH$ = 9/1/0,5) 0,67.

Etape 3

Ester t-butylique de la N[N(benzyloxy) amino carbonyl-3 benzylidène-2 oxo-1 propyl] methyl-2 β alanine

Ce composé est obtenu à partir de 4,5 g du composé précédent et 2,1 g de chlorhydrate de benzylhydroxylamine en suivant le procédé décrit dans l'exemple 1, étape 3. On obtient 4,7 g (Rdt 80 %) d'un produit solide blanc F = 110°C - Rf ($CHCl_3/CH_3OH$ = 9/1) 0,68.

Etape 4

N[N(benzyloxy) aminocarbonyl-3 benzylidène-2 oxo-1 propyl] methyl-2 β alanine

Ce composé est obtenu à partir de 1,3 g du composé précédent en suivant le procédé décrit dans l'exemple 1 étape 4. On obtient 0,49 g d'un produit solide blanc 0,49 g (Rdt 45 %)
F = 119°C - Rf ($CHCl_3/CH_3OH/CH_3COOH$ = 9/1/0,5) 0,62.

Etape 5

N[N(hydroxy) aminocarbonyl-3 benzyl-2 oxo-1 propyl] methyl-2 β alanine

A partir de 0,49 g du composé précédent, on obtient ne suivant le procédé décrit dans l'exemple 1, étape 5, 0,36 g d'un produit solide blanc (Rdt 94 %) F = 88°C -Rf ($CHCl_3/CH_3OH/CH_3COOH$ = 9/1/0,5) 0,18.
Le composé est obtenu sous forme des 4 isomères, lesquels peuvent être séparés, notamment par chromatographie.

**Exemple 4**

Etape 1

Ester t-butylique de la N[benzylidène-2 ethoxy carbonyl-3 oxo-1 propyl] benzyl-2 β alanine

En suivant le procédé décrit dans l'exemple 1, étape 1, on obtient, à partir de 2 g d'acide ethoxy-3 benzylidène-2 propanoïque et de 2 g d'esther t-butylique de la benzyl-2 β alanine, 3,8 g (Rdt 98 %) d'une huile jaune
Rf ($CHCl_3/CH_3OH$ = 9/1) 0,80.

Etape 2

Ester t-butylique de la N[benzylidène-2 carboxy-3 oxo-1 propyl] benzyl-2 β alanine

En suivant le procédé décrit dans l'exemple 1, étape 2, on obtient à partir de 3,8 g du composé précédent, 2,4 g d'un composé huileux (Rdt 67 %)
Rf ($CHCl_3/CH_3OH/CH_3COOH$ = 9/1/0,5) 0,66.

Etape 3

Ester t-butylique de la N[benzyloxy) aminocarbonyl-3 benzylidène-2 oxo-1 propyl] benzyl-2 β alanine

En suivant le procédé décrit dans l'exemple 1, étape 3, on obtient à partir de 2,4 g du composé précédent et 0,9 g de chlorhydrate de benzylhydroxylamine, 2,8 g (Rdt 96 %) d'un produit solide jaune pâle.
F = 67° C, Rf ($CHCl_3/CH_3OH$ = 9/1) = 0,67.

Etape 4

N[N(benzyloxy) amino carbonyl-3 benzylidène-2 oxo-1 propyl] benzyl-2 β alanine

En suivant le procédé décrit dans l'exemple 1, étape 4, on obtient à partir de 1,8 g du composé précédent, 1,17g d'un produit solide blanc (Rdt 74 %)
F 144° C - Rf ($CHCl_3/CH_3OH/CH_3COOH$ = 9/1/0,5) 0,65.

Etape 5

N[N(hydroxy)amino carbonyl-3 benzylidène-2 oxo-1 propyl] benzyl-2 β alanine

En suivant le procédé décrit dans l'exemple 1, étape 5, on obtient à partir de 0,63 g du composé précédent, 0,5g d'un produit solide blanc (Rdt 97 %)
F 86° C - Rf ($CHCl_3/CH_3OH/CH_3COOH$ = 9/1/0,5) 0,30. Le composé est obtenu sous forme des 4 isomères, lesquels peuvent être séparés, notamment par chromatographie.

**Exemple 5**

Etape 1

Ester t-butylique de la N(benzylidène-2 ethoxy carbonyl-3 oxo-1 propyl] benzyl-3 β alanine

En suivant le procédé décrit dans l'exemple 1, étape 1, on obtient à partir de 1,1 g d'acide éthoxycarbonyl-3 benzylidène-2 propanoïque et 1,1 g de l'ester t-butylique de la benzyl-3 β alanine, 1,86 g d'une huile jaune (Rdt 87 %) Rf ($CHCl_3/CH_3OH/CH_3COOH$ = 9/1/0,5) 0,80.

Etape 2

Ester t-butylique de la N[benzylidène-2 carboxy-3 oxo-1 propyl] benzyl-3 β alanine

A partir de 1,86 g du composé précédent, on obtient, en suivant le procédé décrit dans l'exemple 1, étape 2, 1,1 g d'un composé huileux (Rdt 63 %)
Rf ($CHCl_3/CH_3OH$ = 9/1) 0,41.

Etape 3

Ester t-butylique de la N[N(benzyloxy) amino-carbonyl-3 benzylidène=2 oxo-1 propyl] benzyl-3 β alanine

En suivant le procédé décrit dans l'exemple 1, étape 3, par traitement de 1 g du composé précédent et du 0,4 g de chlorhydrate de benzylhydroxylamine, on obtient 1 g d'un produit solide jaune pâle (Rdt 76 %)
F 61°C-Rf ($CHCl_3/CH_3OH$ = 9/1) 0,75.

Etape 4

N[N(benzyloxy) amino carbonyl-3 benzylidène-2 oxo-1 propyl] benzyl-3 β alanine

A partir de 0,48 g du composé précédent, on obtient en suivant le procédé décrit dans l'exemple 1, étape 4, 0,30 g d'un produit solide blanc (Rdt 70 %)
F 115° C-Rf-($CHCl_3/CH_3OH/CH_3COOH$ = 9/1/0,5) 0,62.

Etape 5

N[N(hydroxy) amino carbonyl-3 benzyl-2 oxo-1 propyl] benzyl-3 β alanine

A partir de 0,29 g du composé précédent, on obtient en suivant le procédé décrit dans l'exemple 1, étape 5, 0,22 g (Rdt 94%) d'un produit huileux jaune pâle qui cristallise lentement.

Le deuxième carbone asymétrique ayant la configuration S, le composé obtenu est un mélange de 2 diastéréoisomères lesquels peuvent être séparés, notamment par chromatographie.

Le composé (R,S) a les caractéristiques suivantes :

Rf ($CHCl_3/CH_3OH/CH_3COOH$ = 9/1/0,5) = 0,33 ; F = 138°C.
Le composé (S,S) a les caractéristiques suivantes :
Rf ($CHCl_3/CH_3OH/CH_3COOH$ = 9/1/0,5) = 0,27 ; F = 168°C.

**Exemple 6**

Etape 1

Ester t.butylique de la N[benzylidène-2 éthoxy carbonyl-3 oxo-1 propyl] phényl-2 β alanine

Ce composé est obtenu en suivant le procédé décrit pour le composé 1, étape 1. A partir de 1,3 g de l'acide benzylidène-2 éthoxy carbonyl-4 propanoïque et 1,2 g de l'ester t.butylique de la phényl-2 β-alanine, on obtient 2,2 g d'une huile jaune (Rdt 91 %).
Rf ($CHCl_3/CH_3OH/CH_3COOH$ = 9/1/0,5) 0,76.

Etape 2

Ester t.butylique de la N[benzylidène-2 carboxy-3 oxo-1 propyl] phenyl-2 β-alanine.

Ce composé est obtenu en suivant le procédé décrit dans l'exemple 1, étape 2. A partir de 2,2 g du composé précédent, on obtient 1,27 g (Rdt 61 %) d'une huile jaune pâle.
Rf ($CHCl_3/MeOH/CH_3COOH$ = 9/1/0,5) 0.79.

Etape 3

Ester t.butylique de la N[benzyloxyamino carbonyl-3 benzylidène-2 oxo-1 propyl] phenyl-2 β-alanine.

Ce composé est obtenu en suivant le procédé décrit dans l'exemple 1, étape 3. A partir de 1,2 g du composé précédent on obtient 1,24 g d'un produit solide blanc.
F = 61°C. Rdt 80 % Rf ($CHCl_3/CH_3OH/CH_3COOH$ = 9/1/0,5)0,66.

Etape 4

N[N(benzyloxy amino) carbonyl-3 benzylidène-2 oxo-1 propyl] phényl-2 β alanine.

Ce composé est obtenu en suivant le procédé décrit dans l'exemple 1, étape 4. A partir de 0,64 g du composé précédent, on obtient 0,47 g (82 %) d'un produit solide blanc.
F 132°C. Rf ($CHCl_3/CH_3OH/CH_3COOH$ = 9/1/0,5) 0,67.

Etape 5

N[N-hydroxyamino) carbonyl-3 benzyl-2 oxo-1 propyl] phényl-2 β-alanine.

Ce composé est obtenu en suivant le procédé décrit dans l'exemple 1, étape 5. A partir de 0,42 g du composé précédent, on obtient 0,25 g (72 %) d'un solide blanc. F 178°C. Rf ($CHCl_3/CH_3OH/$-$CH_3COOH$ = 0/1/0,5) 0,38. Le composé est obtenu sous forme des 4 isomères, lesquels peuvent être séparés, notamment par chromatographie.

**Exemple 7**

Etape 1

Ester t.butylique de l'acide N[éthoxy carbonyl-3 benzylidène-2 oxo-1 propyl] amino-2 cyclohexane carboxylique.

Ce composé est obtenu en suivant le procédé décrit dans l'exemple 1, étape 1. A partir de 0,64 g d'acide éthoxy carbonyl-3 benzylidène-2 carboxylique et de 0,71 g d'ester t.butylique de l'acide amino-2 cyclohéxane carboxylique, on obtient 0,82 g (72 %) du produit attendu.
Rf ($CHCl_3/CH_3OH/CH_3COOH$ = 9/1/0,5) 0,86.

Etape 2

Ester t.butylique de l'acide N[benzylidène-2 carboxy-3 oxo-1 propyl] amino-2 cyclohéxane carboxylique.

Ce composé est obtenu en suivant le procédé décrit dans l'exemple 1, étape 2. A partir de 0,82 g du composé précédent on obtient 0,53 g d'une huile jaune (70 %).
Rf ($CHCl_3/CH_3OH/CH_3COOH$) = 9/1/0,5) 0,56.

Etape 3

Ester t.butylique de l'acide N[(benzyloxy aminocarbonyl)-3 benzylidène-2 oxo-1 propyl] amino-2 cyclohéxane carboxylique.

Ce composé est obtenu en suivant le procédé décrit dans l'exemple 1, étape 3. A partir de 0,49 g du composé précédent, on obtient 0,50 g d'une huile jaune (Rdt 80 %).
Rf ($CHCl_3/MeOH/CH_3COOH$ = 9/1/0,5) 0,58.

Etape 4

Acide N[benzyloxy aminocarbonyl)-3 benzylidène-2 oxo-1 propyl] amino-2 cyclohéxane carboxylique

Ce composé est obtenu en suivant le procédé décrit dans l'exemple 1, étape 4. A partir de 0,48 g du composé précédant, on obtient 0,37 g d'un solide blanc (Rdt 85 %).
F 157°C. Rf ($CHCl_3/CH_3OH$ = 9/1) 0,40.

Etape 5

Acide N[(hydroxy amino carbonyl)-3 benzyl-2 oxo-1 propyl] amino-2 cyclohéxane carboxylique.

Ce composé est obtenu en suivant le procédé décrit dans l'exemple 1, étape 5. A partir de 0,20 g du composé précédent, on obtient 0,15 g du produit attendu (Rdt 95 %). Le cyclohexane ayant la configuration cis le composé obtenu est sous forme de 2 diastéréoisomères, lesquels peuvent être séparés, notamment par chromatographie sur gel de silice.

Le composé (R, cis) a les caractéristiques suivantes :
Rf ($CH_3ClEt$/pyridine/$CH_3COOH/H_2O$ = 160/20/6/11)
Le composé (S, cis) a les caractéristiques suivantes :
Rf ($CH_3ClEt$/pyridine/$CH_3COOH/H_2O$ = 160/20/6/11)

**Exemple 8**

Etape 1

(N.t.butyloxycarbonyl)amino-3-phenyl-4(N'benzyloxyamino)-1 oxo-1 butane

A une solution de 530 mg d'acide-N-butyloxycarbonyl amino-3 phenyl-4 butanoïque dans le chloroforme sont ajoutées successivement à 0°C, une solution de 300 mg de chlorydrate de benzylhydroxylamine et de 0,28 ml de triethylamine dans le chloroforme, une solution de 290 mg d'hydroxybenzotriazole dans le THF anhydre et une solution de 390 mg de dicycloherylcarbodiimide dans le chloroforme. On agite 1 heure à 0°C et toute la nuit à température ambiante. Après filtration du dicyclohexylurée (DCU) et évaporation du solvant, le résidu est traité comme dans l'exemple 1, étape 1.

On obtient 380 mg (52 %) d'un produit solide F 98°C -
Rf ($CHCl_3/CH_3OH$ = 9/1).

Etape 2

Trifluoroacetate de l'amino-phenyl-4 (N'-benzyloxyamino)-1 oxo-1 butane

On dissout 370 mg du composé précédent dans 1 ml de $CH_2Cl_2$. On refroidit à 0°C et on ajoute 1 ml de TFA. On agite 1 h à 0°C et 1 h à température ambiante. On évapore à sec et on reprend par l'éther. Un produit solide blanc précipite.
P : 380 mg - Rdt 98 % -
Rf ($CHCl_3/CH_3OH$ = 8/2) 0,45.

Etape 3

[N(benzyloxycarbonyl)-3-oxo-1 propyl) amino]-3 phenyl-4 N'(benzyloxyamino)-1 oxo-1 butane

A une solution de 195 mg (0,49 mM) du composé précédent et de 0,07 ml de triethylamine dans le chloroforme, sont ajoutées successivement à 0°C, une solution de 102 mg (0,49 mmol) de monobenzyl succinate dans le chloroforme, une solution de 75 mg (0,49 mmol) d'hydroxybenzotriazole dans le THF anhydre et une solution de 163 mg (0,59 mmole) de dicylohexylecarbodiimide dans $CHCl_3$. Après traitement dans les conditions standard d'un couplage (exemple 1, étape 1) on obtient 174 mg (77 %) d'un composé huileux purifié par chromotographie sur gel de silice ($CH_2Cl_2/MeOH$ = 7/3).
Rf ($CHCl_3/CH_3OH$ = 0,52.

Etape 4

N[carboxy-3 oxo-1 propyl) amino]-3 phenyl-4 (N'[hydroxyamino]-1 oxo-1 butane;

Une solution méthanolique du composé précédent (105 mg, 0,23 mmole) est hydrogéné à température et pression ordinaires en présence de 27 mg de palladium sur charbon. Après absorption de la quantité théorique d'hydrogène, on filtre le catalyseur et on évapore à sec. On obtient 66 mg (97 %) du produit attendu

solide blanc F = 92°C, Rf ($CHCl_3/CH_3OH/CH_3OH$ = 9/1/0,5) 0,30.
Le composé est obtenu sous forme de composé optiquement pur, dans lequel le carbone asymétrique a la configuration R.

**Exemple 9**

Etape 1

Acide benzyloxycarbonyl-3 methylidène-2 propanoïque

On chauffe à 80°C pendant 4 heures un mélange de 4 g d'anhydride itaconique et 50 ml d'alcool benzylique. On évapore à sec. Résidu huileux 7,5 g (95 %).

Etape 2

N[Benzyloxycarbonyl)-3 methylidène-2 oxo-1 propyl) amino]-3 phenyl-4 N'(benzyloxyamino)-1 oxo-1 butane

A une solution de 500 mg (1,26 mmole) du trifluoroacetate de l'amino-3-phenyl-4 (N-benzyloxy) amino-1 oxo-1 butane et de 0,8 ml de triethylamine dans le chloroforme sont ajoutées successivement à 0°C une solution de 276mg (1,26 mmol) d'acide benzyloxycarbonyl-3 methylidène-2 propanoïque dans le chloroforme, une solution de 192 mg (1,26 mmole) d'HOBT dans le THF anhydre et une solution de 285 mg (1,38 mmole) de DCC dans le chloroforme. Après traitement dans les conditions standard du couplage, on obtient 428 mg (70 %) du composé attendu
Rf ($CHCl_3/CH_3OH$ = 9/1) 0,50.

Etape 3

N[(carboxy-3 methyl-2 oxo-1 propyl) amino]-3 phenyl-4 N'hydroxyamino)-1 oxo-1 butane

Une solution methanolique de 400 mg du composé précédent est hydrogénée à température et pression ordinaire en présence de palladium sur charbon. Après absorption de la quantité théorique d'hydrogène on filtre le catalyseur et on évapore à sec. On obtient 230 mg (F = 130°C).

Le composé est obtenu sous forme de deux diastéréoisomères dans lesquels le premier carbone asymétrique a la configuration R, et qui peuvent être séparés, notamment par chromatographie.

**Exemple 10**

Etape 1

Acide t-butyloxycarbonyl-3 butanoïque

On dissout 850 mg d'acide benzyloxycarbonyl-3 methylidène-2 propanoïque (exemple 7, étape 1), dans 2 ml d'éther et on ajoute à 20°C 20 )l d'acide sulfurique concentré et on sature en isobutylène. Après 24 h, on évapore l'excès d'isobutylène, on reprend le résidu par l'éther et on lave 4 fois par une solution de $NaHCO_3$ à 5 %, une fois à l'eau, une fois avec NaCl saturé puis on sèche sur $Na_2SO_4$. Après évaporation à sec, on obtient une huile 650 mg (60 %)
Rf ($CHCl_3/CH_3OH$ = 9/1) 0,85.

Une solution dans le méthanol du composé précédent (630 mg) est hydrogénée en présence de palladium sur charbon à température et pression ordinaires. Après filtration du catalyseur et évaporation à sec, on obtient 370 mg (86 %) du produit attendu.

Etape 2

N[t-butyloxycarbonyl)-3 oxo-1 butyl) amino]-3 phenyl-4 N'(benzyloxyamino-1 oxo-1 butane

A une solution de 380 mg (0,96 mmole) trifluoroacetate de l'amino-3 phenyl-4 (N-benzyloxy) amino-1 oxo-1 butane et de 0,14 ml de triethylamine dans le chloroforme, on ajoute successivement à 0°C, une solution de 180 mg (0,96 mmole) d'acide t-butyloxycarbonyl-3 butanoïque dans le chloroforme, une solution de 147 mg (0,96 mmole) HOBT dans le THF et 217 mg (1,05 mmole) de DCC dans le chloroforme. Après traitement dans les conditions standard du couplage on obtient 300 mg (69 %) du composé attendu.
Rf ($CHCl_3$/Ether = 50/50) 0,27.

Etape 3

N[carboxy-3 Oxo-1 butyl) amino]-3 phenyl-4 N'(benzyloxyamino)-1 oxo-1 butane

On dissout 150 mg du composé précédent dans 0,6 ml de $CH_2Cl_2$ et on ajoute, à 0°C, 0,6 ml de TFA. On laisse une nuit à 0°C. On évapore à sec et reprend par l'éther. On obtient un solide blanc. 120 mg (91 %)
Rf ($CHCl_3/CH_3OH$ = 9/1) 0,35. F = 150°.

Le composé est obtenu sous forme de deux diastéréoisomères, dans lesquels le premier carbone asymétrique a la configuration R, et qui peuvent être séparés, notamment par chromatographie.

Exemple 11

Etape 1

N[N(benzyloxyamino) carbonyl-1 phenyl-3]-2 carboxamido-1 carbethoxy-2 cyclohéxane.
Ce composé est obtenu en suivant le procédé décrit pour l'exemple 9 étape 2.
A partir de 0,50 g du trifluoroacétate de l'amino-3 phényl-4 N-benzyloxyamino-1 oxo-1 butane et 0,25 g du

**Exemple 11**

Etape 1

N[N(benzyloxyamino) carbonyl-1 phenyl-3]-2 carboxamido-1 carbethoxy-2 cyclohéxane.
Ce composé est obtenu en suivant le procédé décrit pour l'exemple 9 étape 2.
A partir de 0,50 g du trifluoroacétate de l'amino-3 phényl-4 N-benzyloxyamino-1 oxo-1 butane et 0,25 g du monoester éthylique de l'acide cyclohéxane dicarboxylique, on obtient 0,50 g d'un composé huileux (Rdt 85 %).
Rf ($CHCl_3/CH_3OH$ = 9/1) 0,67.

Etape 2

Acide[N[N(benzyloxyamino) carbonyl-1 phényl-3] propyl]-2 carboxamido cyclohexanoïque.
Ce composé est obtenu en suivant le procédé décrit dans l'exemple 1, étape 2. A partir de 0,47 g du composé précédent on obtient 0,25 g d'un produit solide blanc.
F = 60°C (Rdt 56 %).
Rf($CH_2Cl_2/CH_3OH/CH_3COOH$ = 8/2/0,2) 0,56.

Etape 3

Acide [N[N(hydroxyamino) carbonyl-1 phényl-3] propyl]-2 carboxamido-2 cyclohéxanoïque.
Ce composé est obtenu en suivant le procédé décrit dans l'exemple 1, étape 5. A partir de 0,25 g du composé précédent on obtient le produit attendu. Cristaux blancs 0,19 g (Rdt 95 %).
F = 138°C. Rf ($CH_2Cl_2/CH_3OH/CH_3COOH$ = 8/2/0,2) 0,53.
Le composé est obtenu sous forme optiquement pure et dans laquelle le premier carbone asymétrique a la configuration (R) et le cyclohexane a la forme cis.

Les composés de l'invention possèdent d'intéressantes propriétés inhibitrices des trois enzymes qui dégradent l'enképhaline, à savoir l'endopeptidase, l'aminopeptidase et la dipeptidylaminopeptidase, ainsi que des propriétés antalgiques.

**Exemple 12**

Etape 1

(R) N-(carbéthoxy-3 benzylidène-2 oxo-1 propyl) amino-3 phényl-4 N'-(benzyloxy) butanamide
Ce composé est obtenu en suivant le procédé décrit pour l'exemple 9 étape 2. A partir de 0,500 g de trifluoacétate de l'amino-phényl-4-N-benzyloxyamino 1-oxo-butane et de 0,29 g d'acide benzylidène succinique on obtient, après traitement dans les conditions de couplage, un solide blanc (0,58 ; R = 93 %).
PF = 108°C,
Rf : ($CH_2Cl_2$/MeOH = 9/1) = 0,67.

Etape 2

(R) N-(carboxy-3 benzylidène-2 oxo-1 propyl) amino-3 phényl-4 N'-(benzyloxy) butanamide
A partir de 0,56 g du composé précédent, on obtient, dans les mêmes conditions que celles décrites dans l'exemple 1 étape 2, une poudre blanche (0,25 g ; R = 47 %), RF = 145°-150°C.
Rf. ($CH_2Cl_2$/MeOH = 9/1) = 0,78.

Etape 3

(R) N-[(R,S benzyl-2 carboxyl-3 oxo-1 propyl) amino-3 phényl-4 N'-(hydroxy)] butanamide
A partir de 0,190 g du composé précédent, on obtient, par hydrogénation catalytique, une poudre blanche (0,147 g)R = 95,5 %). Les deux diastéréoisomères (notés A et B) ont été séparés sur colonne de silice éluant : AcOEt/Py/AcOH:/$H_2O$ = 180/20/6/11 : RfA = 0,4 ; RfB = 0,23, PFA = 198°C ; PFB = 220°C. CLHP:TFA 0,07/MeCN:71/29 ;

$t_R$ = 7 min. 42 sec. (RS):14 min. (RR).

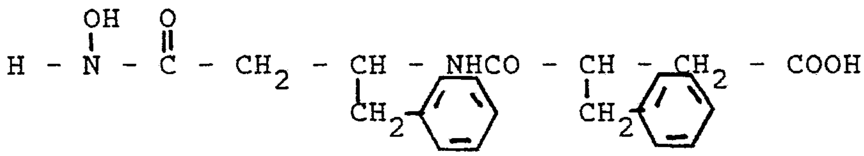

**Exemple 13**

Etape 1

Ester α-tert butylique β-éthylique de l'acide benzylidène succinique

1 g de monoester éthylique (β) de l'acide benzylidène succinique est traité par un excès d'isobutylène dans l'éther (5 ml) en présence de $H_2SO_4$ (0,025 ml). Une huile jaune pâle est obtenue (995 mg : R = 80 %). Rf : ($CHCl_3$/MeOH = 9/1).

Etape 2

Ester α-tert butylique de l'acide benzylidène succinique

A partir de 859 mg du composé précédent, on obtient, après traitement dans les conditions décrites dans l'exemple l'étape 2, une poudre crème (862 mg : R = 90 %). Rf : ($CHCl_3$/MeOH = 9/1). 0,51 PF 104°C.

Etape 3

(R) N-(t-butyloxycarbonyl-3 oxo-1 phényl-4 butène-3 yl) amino-3 phényl-4 N'(benzyloxy) butanamide

A partir de 500 mg du trifluoroacétate de l'amino-3 phényl-4 N(benzyloxyamino-1 oxo-1) butane et de 329 mg du composé précédent, on obtient, par traitement dans les conditions de couplage, une poudre blanche (517 mg/R = 78 %). PF = 58°C. Rf : ($CH_2Cl_2$/MeOH = 9/1) = 0,70.

Etape 4

(R) N-(carboxy-3 oxo-1 phényl-4 butène-3 yl) amino-3 phényl-4 N'-(benzyloxy) butanamide

260 mg du composé précédent sont traités par l'acide trifluoroacétate. Une poudre blanche est obtenue (219 mg, R = 94 %). PF = 174°-176°C. Rf:($CH_2Cl_2$/MeOH/AcOH = 9/1,5/0,3) = 0,54.

Etape 5

(R) N-[(R,S) carboxy-3 oxo-1 phényl-4 butyl) amino-3 phényl-4 N'-(hydroxy)] butanamide

A partir de 200 mg du composé précédent, on obtient par hydrogénation catalytique une poudre blanche (154 mg:R = 95 %). PF = 105°C. Rf : ($CH_2Cl_2$/MeOH/AcOH = 9/0,5/0,3). = 0,17. Les deux diastéréoisomères (A et B) sont séparés sur colonne de silice : solvant d'élution : (AcOEt/Py/AcOH/$H_2O$ : 180/20/6/11). Rf A = 0,23:Rf B = 0,17. CLHP:TFA 0,07 %/MeCN:71/29. $t_R$ = 9 min. 24 sec. et 11 min. 48 sec.

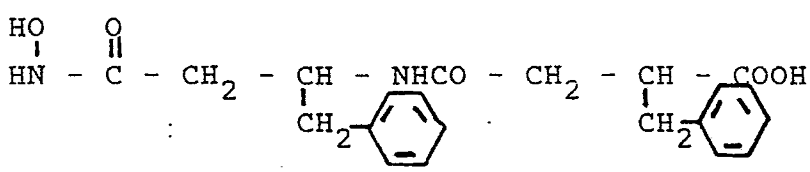

Exemple 14

Etape 1

(R) N-(méthoxycarbonyl-2 (trans) c-pentanoyl)amino-3 phényl-4 N'-(benzyloxy) butanamide

A partir de 500 mg du trifluoroacétate de l'amino-3 phényl-4 N-benzyloxyamino-1 oxo-1 butane et de 216 mg du monoester méthylique de l'acide trans cyclopentane 1,2 dicarboxylique, on obtient, après traitement dans les conditions de couplage, un solide blanc (416 mg ; R = 75,6 %). PF = 128°C.
Rf : ($CH_2Cl_2$/MeOH = 9/1). = 0,60.

Etape 2

(R) N-(carboxy-2 (trans) c-pentanoyl) amino-3 phényl-4, N'-(benzyloxy) butanamide

A partir de 390 mg du composé précédent dissous dans le méthanol (9 ml), on obtient, après traitement dans les conditions décrites pour l'exemple 1 étape 2, une poudre blanche (307 mg/R = 81,4 %). PF = 153°C.
Rf : ($CHCl_3$/MeOH/AcOH = 9/1/0,5). = 0,51

Etape 3

(R) N-(carboxy-2(trans) c-pentanoyl) amino-3 phényl-4 N'-(hydroxy) butanamide 49

A partir de 255 mg (0,6 mmole) du composé précédent, on obtient après hydrogénation catalytique, une poudre blanche que l'on recristallise (MeOH/$H_2O$)(1,85 mg ; R = 92 %). PF = 142°C.
Rf : (AcOEt/Py/AcOH : $H_2O$ = 180/20/9/11) = 0,36 et 0,26. CLHP : TFA 0,07 %/MeCN : 75/25. $t_R$ = 5 min. 01 sec. et 7 min. 54 sec.

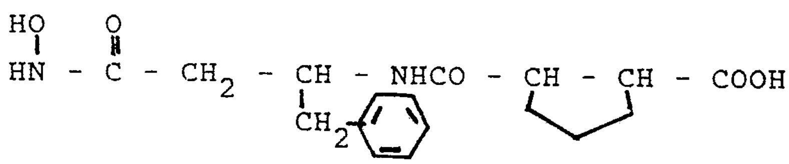

**Exemple 15**

Etape 1

Ester t-butylique de l'acide N [éthoxy carbonyl-3 benzylidène-2-oxo 1 propyl] amino 2 cyclopentane carboxylique

Ce composé est obtenu en suivant le procédé dans l'exemple 1 étape 1. A partir de 1,42 g de l'acide éthoxy carbonyl-3 benzylidène-2 carboxylique et de 1,39 g de l'ester t-butylique de l'acide amino 2 cyclopentane carboxylique, on obtient 1,63 g (67 %) du produit attendu.
Rf : ($CHCl_3$/MeOH 9/1) 0,83

Etape 2

Ester t-butylique de l'acide N [benzylidène-2 carboxy-3-oxo-1 propyl] amino 2 cyclopentane carboxylique

Ce composé est obtenu en suivant le procédé décrit pour l'exemple 1, étape 2. A partir de 1,6 g du composé précédent, on obtient 0,90 g (60 %) d'une huile jaune pâle.
Rf : ($CHCl_3$/MeOH/$CH_3COOH$ = 9/1/0,5) 0,67.

Etape 3

Ester t butylique de l'acide N[benzy(oxyaminocarbonyl)-3 benzylidène-2-oxo-1 propyl] amino-2 cyclopentane carboxylique

Ce composé est obtenu en suivant le procédé décrit dans l'exemple 1 étape 3. A partir de 0,86 g du composé précédent, on obtient 0,98 g d'un solide blanc F 112°C (89 %).
Rf : ($CHCl_3$/MeOH = 9/1) = 0,64.

Etape 4

Acide N(benzyloxy amino carbonyl)-3 benzylidène-2-oxo-1 propyl] amino-2 cyclopentane carboxylique

Ce composé est obtenu en suivant le procédé décrit dans l'exemple 1 étape 4. A partir de 0,61 g du composé précédent, on obtient 0,44 g d'un solide blanc, F 168°C, Rdt 82 %.
Rf : ($CHCl_3$/MeOH = 9/1) = 0,30.

Etape 5

Acide N[(hydroxy aminocarbonyl)-3 benzyl-2-oxo-1-propyl] amino-2 cyclopentane carboxylique

Ce composé est obtenu en suivant le procédé décrit dans l'exemple 1 étape 5. A partir de 0,43 g du composé précédent, on obtient 0,33 g du composé final (Rdt. 97 %).
Le cyclopentane ayant la configuration trans, le produit obtenu est un mélange de deux diastéréoisomères, qui peuvent être séparés par chromatographie sur gel de silice.

Composé A. Rf : ($CHCl_3$/MeOH/AcOH = 9/1/0,2) = 0,47
Composé B. Rf : ($CHCl_3$/MeOH/AcOH = 9/1/0,2) = 0,24.

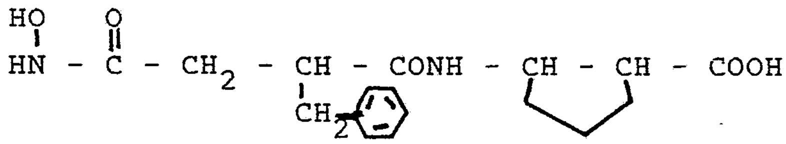

La présente invention a donc encore pour objet un médicament ayant notamment des propriétés inhibitrices de l'endopeptidase, de l'aminopeptidase et de la dipeptidylaminopeptidase et des propriétés antalgiques, caractérisé en ce qu'il contient, à titre de principe actif, un composé de formule I ou un sel d'addition avec un acide ou une base pharmaceutiquement acceptable de ce composé.

On donne ci-après les résultats des études biologiques et pharmacologiques.

I - ETUDE BIOLOGIQUE

1°) Pouvoir inhibiteur sur l'enképhalinase (endopeptidase)

Le pouvoir inhibiteur est déterminé sur de l'enképhalinase purifiée du rein de lapin en suivant le protocole décrit dans la littérature (Llorens, C., B. Malfroy, J.C. Schwartz, G. Gacel, B.P. Roques et al., 1982, J. Neurochem. 39, 1081).

Après une pré-incubation de 15 minutes à 25° C, une aliquote de protéines est incubée pendant 20 minutes à 25° C en présence de 20 nanomoles de [$^3$H]D-Ala$^2$-Leu$^5$ enképhaline et du produit à tester dans le tampon Tris-HCl (pH 7.4) 50 mM. On arrête la réaction par addition d'HCl 0.2 N. Le métabolite tritié [$^3$H]Tyr-D-Ala-Gly est séparé de la D-Ala$^2$-Leu$^5$-enképhaline par chromatographie sur colonne de porapak.

2°) Pouvoir inhibiteur sur l'aminopeptidase

Le pouvoir inhibiteur est mesuré sur l'aminopeptidase purifiée du rein de lapin en suivant le protocole décrit dans la littérature (Romaine Bouboutou, Gilles Waksman, Joceyline Deim, Marie-Claude Fournié-Zaluski and Bernard P. Roques, Life Sciences, vol. 35, pp. 1023-1030, 1984).

Le dosage est conduit d'une manière identique au précédent, en utilisant la [$^3$H]Leu$^5$-enképhaline à la place de la [$^3$H]D-Ala$^2$-Leu$^5$-enképhaline comme substrat.

3°) Pouvoir inhibiteur sur la dipeptidylaminopeptidase

Le pouvoir inhibiteur est mesuré sur la dipeptidylaminopeptidase purifiée à partir du cerveau de rat en suivant le protocole décrit (Romaine Bouboutou, Gilles Waksman, Joceyline Deim, Marie-Claude Fournié-Zaluski and Bernard P. Roques, Life Sciences, vol. 35, pp. 1023-1030, 1984).. On utilise également la [$^3$H]Leu$^5$-enképhaline comme substrat.

L'activité des différents produits est exprimée en concentrations inhibitrices 50 %.

Les résultats sont rassemblés dans le tableau I ci-après :

TABLEAU I

| Composés | Enképhalinase(M) | Aminopeptidase(M) | Dipeptidylaminopeptidase(M) |
|---|---|---|---|
| 1 | $2.10^{-9}M$ | $5.10^{-7}M$ | $3.10^{-8}M$ |
| 2 | $5.10^{-9}M$ | $3.10^{-7}M$ | $0,5.10^{-9}M$ |
| 3 | $2.10^{-9}M$ | $4.10^{-7}M$ | $2.10^{-8}M$ |
| 4 | $1,5.10^{-9}M$ | $7.10^{-8}M$ | $1.10^{-9}M$ |
| 5 | $2.10^{-9}M$ | $2.10^{-8}M$ | $0,5.10^{-9}M$ |
| 6 | $1,5.10^{-9}M$ | $8.10^{-8}M$ | $1.10^{-9}M$ |
| 7 | $4.10^{-9}M$ | $5.10^{-8}M$ | $1,5.10^{-9}M$ |
| 8 | $1,4.10^{-9}M$ | $1.10^{-5}M$ | $8.10^{-7}M$ |
| 9 | $0,9.10^{-9}M$ | $1.10^{-5}M$ | $1,8.10^{-7}M$ |
| 10 | $0,5.10^{-9}M$ | $4.10^{-6}M$ | $8.10^{-7}M$ |
| 11 | $0,5.10^{-9}M$ | $7.10^{-8}M$ | $2.10^{-9}M$ |

Remarque

Le composé 1 est testé sous forme du mélange de ses 2 énantiomères,
Le composés 2, 3, 4 et6 sont testés sous forme du mélange de leurs 4 isomères,
Le composé 5 est testé sous la configuration (R,S),
Le composé 7 est testé sous la configuration (R,cis),
Le composé 8 est testé sous la configuration R,
Les composés 9 et 10 sont testés sous forme du mélange des deux diastéréosomères, dans lesquels la configuration du premier carbone asymétrique est R,
Le composé 11 est testé sous la configuration (R,cis).

II- ETUDE PHARMACOLOGIQUE

a/ Les composés de l'invention sont non toxiques.
b/ Activité antalgique.

1°/ test de la plaque chauffante

Il concerne le réflexe de lèchement et de saut de la souris sur une plaque chauffée à 55° C selon la méthode de Jacob et coll. (Arch. Int. Pharmacodyn. 122, 287-300, 1959 : 133, 296-300, 1961).

Administrés par voie intracérébroventriculaire chez la souris, les inhibiteurs testés présentent un effet propre à la fois sur le temps de latence du saut et sur celui des lèchements. Ces effets sont reversés par la naloxone (antagoniste morphinique).

Les résultats du test de la plaque chaude sont rassemblés dans le tableau II ci-après :

TABLEAU II

| Composés (50 )g) | Saut* (s) | Lèchements** (s) |
|---|---|---|
| 4 | 240 | 20,6 |
| 7 | 240 | 26,2 |
| 11 | 240 | 30 |
| contrôles | 50>10 | 7>2 |

* cut off 240 s ; ** cut off 30 s.

Le cut off correspond au moment où la souris est enlevée si elle n'a pas sauté ;

2°) test du retrait de la queu (tail flick)

Administrés par voie intracerebroventriculaire chez la souris, les composés testés produisent un allongement significatif du temps de retrait de la queue. Ces effets sont renversés par la naloxone.

Les résultats figurent dans le tableau III ci-après :

TABLEAU III

| Composés (75 )g) | temps de latence* (s) |
|---|---|
| 4 | 15>4 |
| 11 | 21>3 |
| 7 | 19>5 |
| contrôles | 8>2 |

* cut off 25s.

3°) test d'analgésie sur tissus enflammés (technique de Randall et Selitto).

Test de Randall et Selitto. (Arch. Int. Pharmacodyn. 1957, CXI, 409-419).

L'inflammation accroît la sensibilité à la douleur, notamment à la pression, et les analgésiques élèvent le seuil de cette sensibilité.

La technique consiste à rechercher l'effet analgésique chez le rat dont le seuil de sensibilité a été abaissé par inflammation des articulations. Administrés par voie i.v., les inhibiteurs testés produisent des effets analgésiques très importants réversés par la naloxone. Les effets maximum sont obtenus 20 minutes après l'injection.

Les résultats figurent dans le tableau IV ci-après :

TABLEAU IV

| Composés(5 mg/kg) | % augmentation seuil de focalisation |
|---|---|
| contrôle | 113>4 |
| 4 | 528>5 |
| 7 | 432>5 |

Les résultats de cette étude mettent en évidence les intéressantes propriétés antalgiques et analgésiques de ces inhibiteurs qui les rendent utiles en médecine humaine et vétérinaire.

Les médicaments, objet de l'invention, peuvent être utilisés dans le traitement des algies musculaires, articulaires ou nerveuses, des douleurs dentaires, des zonas et des migraines, ainsi que dans le traitement des affections rhumatismales et aussi à titre de traitement complémentaire dans les états infectieux et fébriles.

L'invention s'étend aux compositions pharmaceutiques renfermant comme principe actif, les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecin humaine comme, par exemple, les comprimés simples ou dragéifiés, les gélules, les granulés, les suppositoires, les ampoules buvables, les solutés injectables, les pommades, les crèmes, les gels et les préparations en aérosols ; elles sont préparées selon les méthodes usuelles. Le principe actif peut y être incorporé à des excipients et adjuvants habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie varie notamment en fonction de la voie d'administration, de l'affection traitée et du sujet en cause.

Par exemple, chez l'adulte, elle peut être conditionnée pour être administrée à des doses d'environ 20 mg à environ 2 g de substance active par jour.

Les compositions pharmaceutiques de l'invention se présentent avantageusement sous forme de dose unitaire constituée par des comprimés ou des dragés contenant environ 1 à environ 50 mg de substance active.

Les compositions de l'invention se présentent avantageusement sous forme de dose unitaire constituée par une solution buvable pour un dosage unitaire d'environ 1 à environ 50 mg de substance active.

**Revendications**

1. Composés de formule générale :

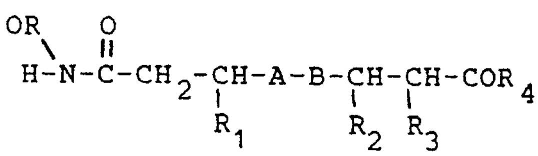

(I)

dans laquelle :
R est
- un atome d'hydrogène ;
- un groupe acyle aliphatique, linéaire ou ramifié, de 2 à 11 atomes de carbone et de préférence de 2 à 5 atomes de carbone, dans lequel 1 atome d'hydrogène peut être substitué par
* un halogène, notamment le fluor, ou un groupe trihalogènométhyle,
* un groupe $OR_5$, $R_5$ représentant l'hydrogène, un groupe alcoyle de 1 à 6 atomes de carbone ou un groupe acyle, de 2 à 7 atomes de carbone, lesquels groupes alcoyle ou acyle peuvent être cyclisés, aliphatiques ou aromatiques ;
- un groupe benzoyle ou phényle acétyle, pouvant comporter sur le cycle aromatique
* 1 à 5 atomes d'halogène, notamment de fluor,

* un groupe $OR_5$, $R_5$ représentant l'hydrogène ou un groupe alcoyle, de 1 à 6 atomes de carbone, ou acyle, de 2 à 7 atomes de carbone, lesquels groupes alcoyle ou acyle peuvent être cyclisés ou aliphatiques ou aromatiques ;
* un groupe nitro,
* un groupe amino, éventuellement mono ou disubstitué par un radical alcoyle aliphatique ou cyclisé de 1 à 6 atomes de carbone ;
- un groupe α-amino acylé

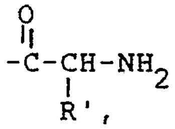

dans lequel
R′ représente le radical benzyle, p-hydroxybenzyle, guanidino-3 propyle ou amino-4-butyle,
$R_1$ représente
- un groupe alcoyle saturé, linéaire ou ramifié, comportant de 1 à 10 atomes de carbone, notamment de 1 à 6 atomes de carbone ;
- un groupe methyl cycloalkyle de 5 à 6 atomes de carbone, un groupe benzyle, les groupes benzyle ou méthyl cycloalcoyle, comportant éventuellement
* 1 à 5 atomes d'halogène, notamment de fluor,
* un hydroxyle,
* un groupe alcoxy, linéaire ou ramifié, comportant de 1 à 4 atomes de carbone
* un groupe amino, éventuellement mono ou disubstitué par un groupe alcoyle aliphatique de 1 à 6 atomes de carbone, éventuellement cyclisé ;
$R_2$ et $R_3$ sont identiques ou différents et représentent
- un atome d'hydrogène ;
- un groupe alcoyle saturé, linéaire ou ramifié, de 1 à 10 atomes de carbone notamment de 1 à 6 atomes de carbone,
- un groupe phényle ou benzyle, pouvant compor ter
* 1 à 5 atomes d'halogène, notamment de fluor,
* un groupe $OR_5$, $R_5$ représente l'hydrogène ou un groupe alcoyle linéaire ou ramifié, de 1 à 5 atomes de carbone, ou un groupe acyle, linéaire ou ramifié de 2 à 5 atomes de carbone,
* un groupe alcoyle aliphatique, linéaire ou ramifié comportant de 1 à 4 atomes de carbone ;

$R_2$ et $R_3$ peuvent être des chaînes alcoyle saturées aliphatiques comportant de 1 à 10 atomes de carbone, notamment de 1 à 6 atomes de carbone, ou des chaînes alcoyle insaturées, comportant de 2 à 10 atomes de carbone, notamment de 2 à 6 atomes de carbone, dans lesquelles 1 ou 2 atomes de carbone peuvent être substitués par des atomes d'oxygène, de soufre ou d'azote, pouvant conduire à la formation de cycles hydrocarbonés saturés ou aromatiques de 5 ou 6 maillons, ou d'hétérocycles saturés ou aromatiques de 5 ou 6 maillons,
$R_4$ représente
- un groupe hydroxyle,
- un groupe alcoxy aliphatique, linéaire ou ramifié, comportant de 1 à 20, avantageusement de 1 à 8, et de préférence de 1 à 4 atomes de carbone, saturé ou insaturé, dans lequel 1 atome de carbone peut être substitué par
* un atome d'halogène, notamment le fluor, ou un groupe trihalogènométhyle,
* un groupe hydroxy,
* un groupe amino, pouvant être mono ou disubstitué par un groupe alcoyle aliphatique de 1 à 6 atomes de carbone, éventuellement cyclisé
* par un groupe aminoxyde 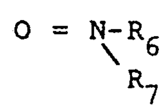

dans lequel $R_6$ et $R_7$ représentent un groupe alcoyle aliphatique de 1 à 6 atomes de carbone, éventuellement cyclisé,
- un groupe $-O-(CH_2)_n-R_5$, n allant de 1 à 4, $R_5$ représentant un cycle saturé de 4 à 6 atomes de carbone ;
- un groupe phényloxy ou phénylalcoyloxy de 1 à 4 atomes de carbone, dans lequel le noyau phényle comporte éventuellement
* 1 à 5 atomes d'halogène, notamment de fluor ;
* un groupe hydroxy,
* alcoxy, dans lequel le radical alcoyle comporte de 1 à 4 atomes de carbone,
* trihalogènomethyle ;

* un groupe nitro, ou amino ;
- un groupe amino, non substitué ou mono ou disubstitué
* par un groupe alcoyle de 1 à 8 atomes de carbone, linéaire ou ramifié,
* par un groupe hydroxyalcoylé de 1 à 8 atomes de carbone,
* par un phényle ou phénylalcoyle dans lequel le groupe alcoyle comporte de 1 à 4 atomes de carbone,
- un reste aminoacide 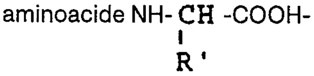

dans lequel R′ représente le radical benzyle, p.hydroxybenzyle, guanidino-3 propyl et amino-4 butyle ;
A-B représente un groupe amide CO-NH ou retro amide NH-CO,
leur mélange racémique, ou leur forme d'énantiomères, de diastéréoisomères, de stéréoisomères et leurs sels formés à l'aide d'une base.

2. Composés de formule I, dans lesquels A-B représente CO-NH et répondant à la formule $I_A$ :

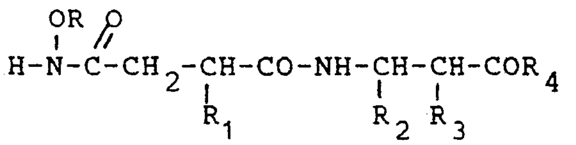 $(I_A)$

dans laquelle R, $R_1$, $R_2$, $R_3$ et $R_4$ ont les significations indiquées à la revendication 1.

3. Composes de formule I, dans lesquels A-B représente NH-CO, et répondent à la formule $I_B$ :

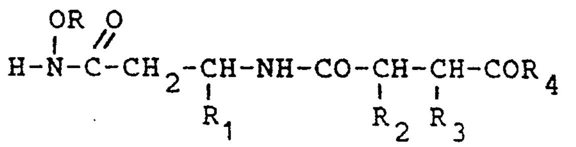 $(I_B)$

dans laquelle R, $R_1$, $R_2$, $R_3$ et $R_4$ ont les significations indiquées à la revendication 1.

4. Composés selon la revendication 1 dans lesquels $R_1$ est choisi parmi

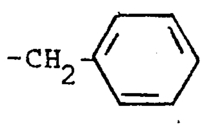 ou 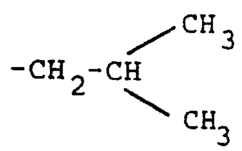

5. Composés selon les revendications 1 à 4, dans lesquels R est choisi dans le groupe constitué par l'hydrogène,

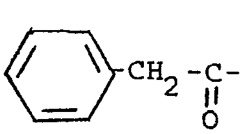 ou 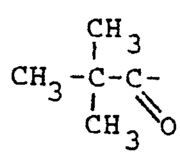

6. Composés selon les revendications 1 à 5, dans lesquels $R_4$ représente un groupe hydroxy, ou un groupe alcoxy aliphatique, linéaire ou ramifié, comportant de 1 à 20, avantageusement de 1 à 8, et de préférence de 1 à 4 atomes de carbone, saturé ou insaturé, dans lequel 1 à 3 atomes de carbone peuvent être substitués par un groupe hydroxy.

7. Composés selon les revendications 1 à 6, dans lesquels l'un au moins des substituants $R_2$ ou $R_3$ est différent de l'hydrogène.

8. Composés selon les revendications 1 à 7, dans lesquels $R_2$ et $R_3$ sont choisis dans le groupe constitué

par H, $CH_3$, 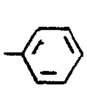 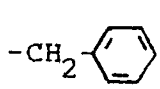 , ou bien $R_2$ et $R_3$ forment

avec les deux carbones auxquels ils sont respectivement fixés un cycle aliphatique saturé de 5 à 6

chaînons.

9. Composés selon l'une des revendications 1 à 8, caractérisé en ce que la configuration du premier carbone asymétrique est (R).

10. Composés selon la revendication 1, de formule

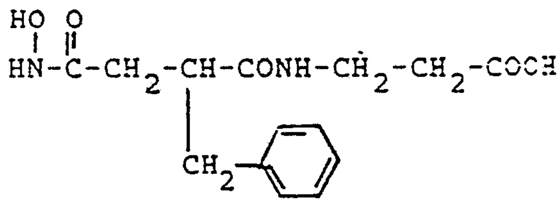

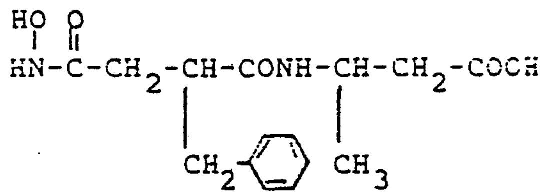

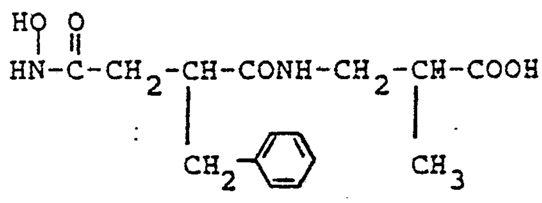

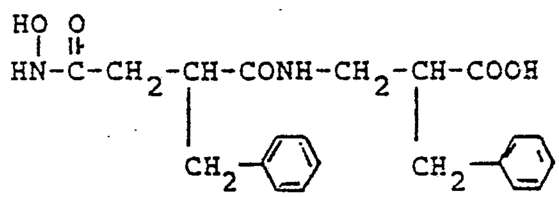

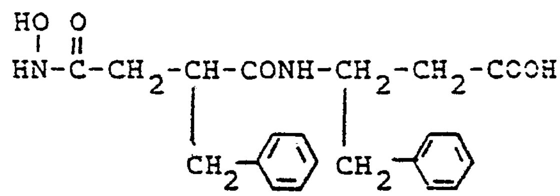

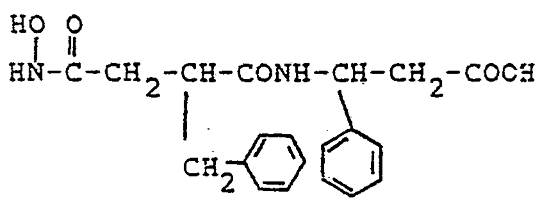

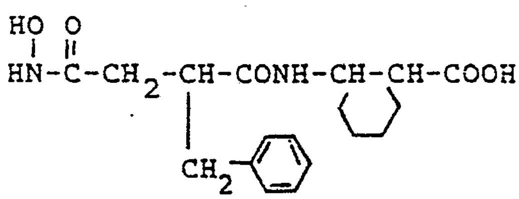

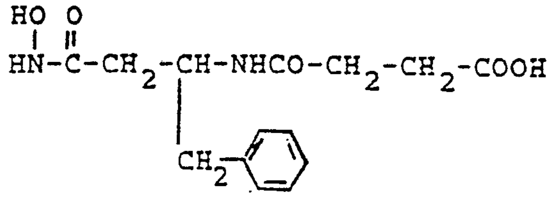

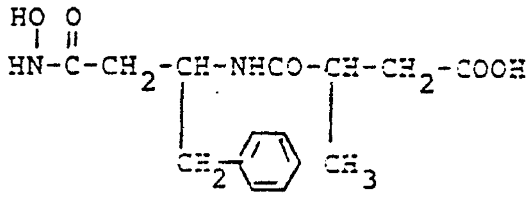

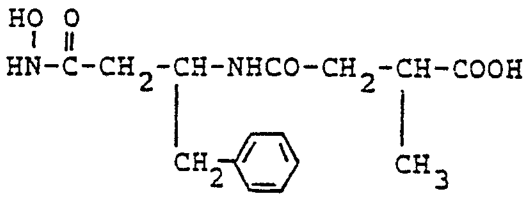

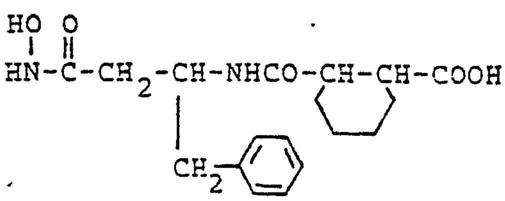

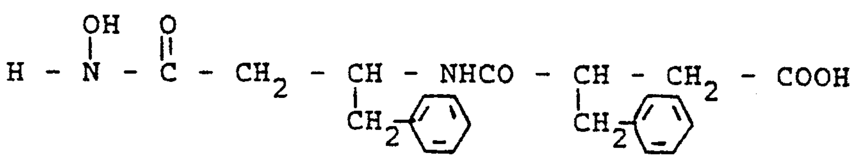

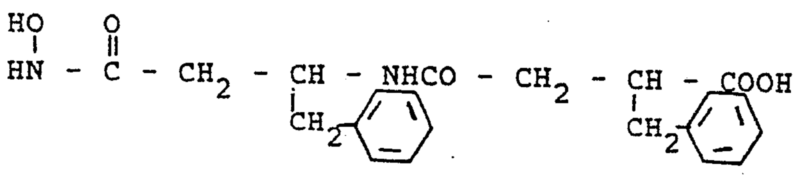

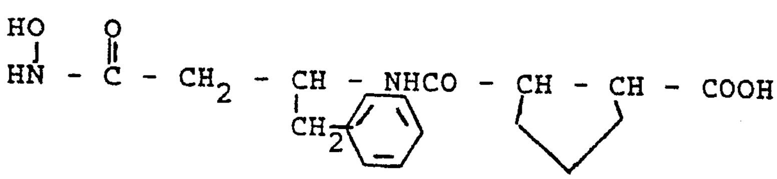

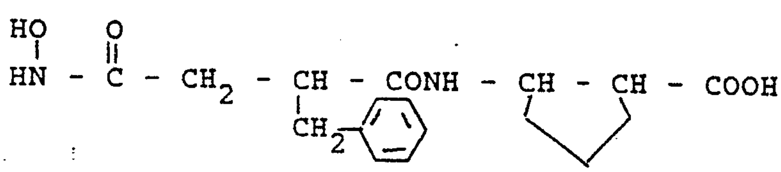

11. Procédé de préparation des composés de formule I, dans lesquels A-B représente une liaison peptidique CO-NH, caractérisé en ce que

- soit on fait réagir le composé de formule II

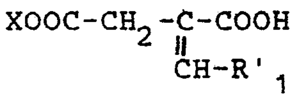

(II)

dans laquelle X représente un groupe alcoyle de 1 à 2 atomes de carbone, notamment $C_2H_5$,
$R'_1$ représente
- un groupe alcoyle saturé, linéaire ou ramifié, comportant de 1 à 9 atomes de carbone, notamment de 1 à 5 atomes de carbone ou un groupe alcoyle insaturé, linéaire ou ramifié, comportant de 2 à 9 atomes de carbone, notamment de 2 à 5 atomes de carbone ;
- un groupe cycloalkyle de 5 à 6 atomes de carbone, un groupe phényle, les groupes phényle ou cycloalcoyle, comportant éventuellement 1 à 5 substitutions par
* un atome d'halogène, notamment le fluor,
* un hydroxyle,
* un groupe alcoxy, linéaire ou ramifié, comportant de 1 à 4 atomes de carbone
* un groupe amino, éventuellement mono ou disubstitué par un groupe alcoyle aliphatique de 1 à 6 atomes de carbone, éventuellement cyclisé ;
sur le composé de formule III

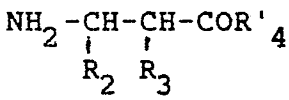

(III)

dans lequel $R'_4$ représente un groupe amide ou un ester tertiobutylique, $R_2$ et $R_3$ ont les significations indiquées à la revendication 1,
pour obtenir le composé de formule IV

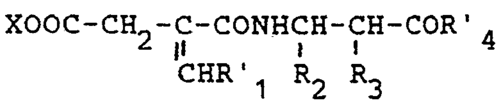

(IV)

en utilisant notamment la dicyclohexylcarbodiimide et l'hydroxybenzotriazole,
- on transforme ensuite le composé IV notamment par hydrolyse alcaline, en composé de formule V

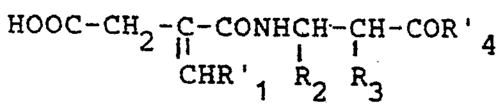

(V)

- on condense ensuite le composé de formule V sur le composé de formule VI

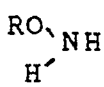

(VI)

dans lesquels R a la signification indiquée à la revendication 1, pour obtenir le composé de formule VII

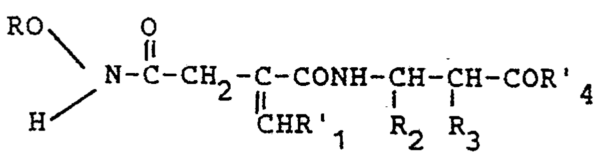

(VII)

en utilisant notamment la dicyclohexylcarbodiimide et l'hydroxybenzotriazole,
- puis, on effectue une hydrogénation catalytique sur le composé VII, pour obtenir le composé de formule $I_{A'}$

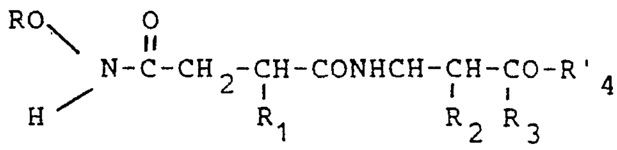

$(I_A)'$

correspondant à $I_A$ si $R_4$ a la même signification que $R'_4$,
- et lorsque l'on souhaite obtenir un composé de formule

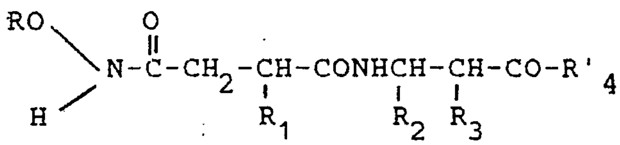

$(I_A')$

dans laquelle $R_4$ est différent de $R'_4$, c'est-à-dire $R_4$ est différent d'un groupe amide et d'un ester tertiobutylique,
* soit on soumet le composé de formule VII

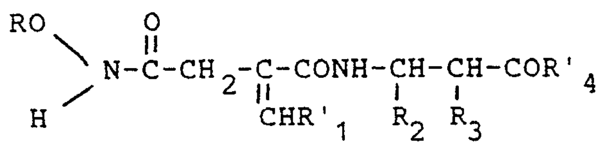

(VII)

dans lequel $R'_4$ représente un ester tertiobutylique, à une hydrolyse acide, pour obtenir le composé de formule VIIbis, ci-dessous indiquée, dans lequel $R_4$ = OH,
* soit on soumet le composé de formule VII indiquée ci-après

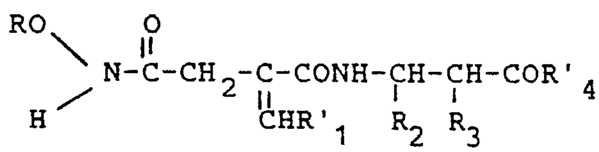

(VII)

dans lequel $R'_4$ représente un ester tertiobutylique, à une hydrolyse acide, suivie d'une reestérification pour obtenir le composé de formule VII bis

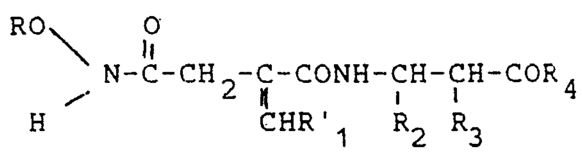

(VII)bis

puis on soumet le composé VII bis obtenu ci-dessus à une hydrogénation catalytique pour obtenir le composé de formule

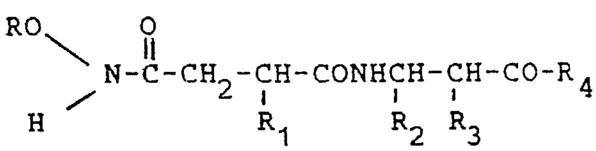

$(I_A)$

* soit on soumet le composé de formule $I'_A$

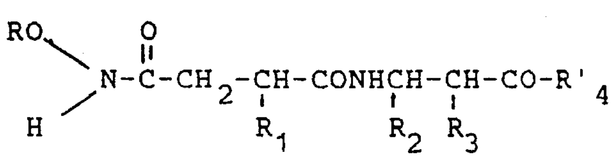

$(I'_A)$

dans lequel $R'_4$ représente un ester tertiobutylique, à une hydrolyse acide, pour obtenir le composé de

formule $I_A$ dans lequel $R_4$ = OH,
* soit on soumet le composé de formule

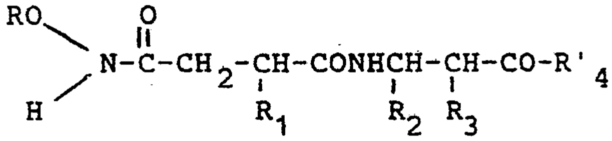

($I_A$')

dans lequel $R'_4$ représente un ester tertiobutylique, à une hydrolyse acide suivie d'une reestérification pour obtenir le composé de formule

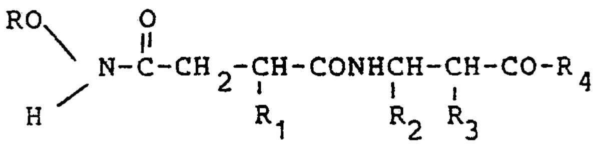

($I_A$)

12. Procédé de préparation des composés de formule I, dans lesquels A-B représente NH-CO, caractérisé en ce que
- on fait réagir le composé de formule VIII

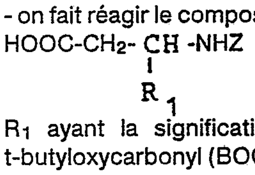

(VIII)

$R_1$ ayant la signification indiquée à la revendication 1, et dans lequel Z représente le groupe t-butyloxycarbonyl (BOC) ou benzyloxycarbonyle sur le composé de formule

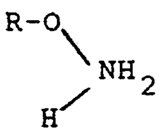

(VI)

R ayant la signification indiquée à la revendication 1, notamment en utilisant de la dicyclohexylcarbodiimide et de l'hydroxybenzotriazole, pour obtenir le composé de formule IX

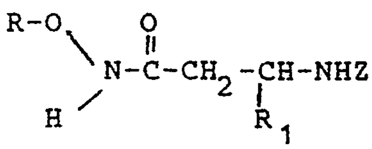

(IX)

- puis on déprotège l'amine en éliminant le groupe Z, par exemple à l'aide d'acide trifluoroacétique, lorsque Z représente BOC et à l'aide d'hydrogénation catalytique lorsque Z représente le groupe benzyloxycarbonyle, pour obtenir le composé de formule X

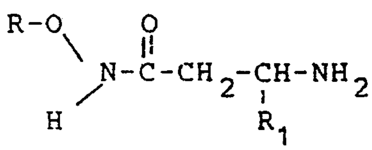

(X)

- puis on fait réagir sur le composé X le composé de formule XI

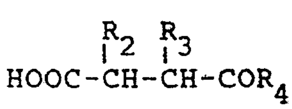

(XI)

$R_2$, $R_3$ et $R_4$ ayant les significations précédemment indiquées, notamment à l'aide de dicyclohexylcarbodiimide et d'hydroxybenzotriazole, pour donner le composé de formule

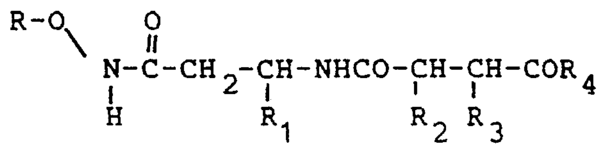

($I_B$)

13. Composition pharmaceutique, caractérisée en ce qu'elle contient comme substance active l'un au moins des composés selon les revendications 1 à 10, en association avec un véhicule pharmaceutiquement acceptable.

14. Composition pharmaceutique, selon la revendication 13, présentant une activité analgésique, caractérisée en ce qu'elle est conditionnée pour être administrée à des doses d'environ 20 mg à environ 2 g de substance active par jour.

15. Composition pharmaceutique, selon la revendication 13, caractérisée par le fait qu'elle se présente sous forme de dose unitaire constituée par des comprimés ou des dragées contenant d'environ 1 à environ 50 mg de substance active.

16. Composition pharmaceutique, selon la revendication 13, caractérisée par le fait qu'elle se présente sous forme de dose unitaire constituée par une solution buvable pour un dosage unitaire de 1 à 50 mg de substance active.

17. Application des composés selon les revendications 1 à 10, pour la préparation de médicaments destinés au traitement d'algies, de douleurs et d'états infectieux fébriles.